# EUROPEAN PATENT APPLICATION

(11) **EP 1 870 131 A1**
(43) Date of publication of application: **26.12.2007**
(21) Application number: 06731588.7
(22) Date of filing: 11.04.2006
(51) Int. Cl.: A61N 1/39

(54) **VENTRICULAR FIBRILLATION PREVENTING DEVICE AND VENTRICULAR FIBRILLATION REMOVING DEVICE**

(30) Priority: 12.04.2005 JP 2005115152; 12.04.2005 JP 2005115153
(71) Applicant: National University Corporation Nagoya University, Nagoya-shi, Aichi 464-8601 (JP); Kyoto University, Kyoto-shi, Kyoto 606-8501 (JP); Japan Health Sciences Foundation, Chuo-ku, Tokyo 103-0001 (JP); Sakuma, Ichiro, Yokohama-shi Kanagawa 240-0045 (JP)
(72) Inventor: SAKUMA, Ichiro, Yokohama Kanagawa 2400045 (JP); KAMIYA, Kaichiro, National University Corporation, Nagoya-shi, Aichi 4648601 (JP); HONJO, Haruo, c/o National University Corporation, Nagoya-shi, Aichi 4648601 (JP); YAMAZAKI, Masatoshi, National University Corporat., Nagoya-shi, Aichi 4648601 (JP); ASHIHARA, Takashi, c/o Graduate School of Medicine, Kyoto-shi, Kyoto 6068501 (JP); NAKAZAWA, Kazuo c/o National Cardiovascular Center, Osaka 5658565 (JP); KODAMA, Itsuo c/o National University Corp., Nagoya-shi Aichi 4648601 (JP)
(74) Representative: TBK-Patent
(86) International application number: PCT/JP2006/307641
(87) International publication number: WO 2006/109797

(57) **Abstract**

[PROBLEMS] To prevent an occurrence of ventricular fibrillation

[MEANS FOR SOLVING PROBLEMS] A rectangular substrate 10 is sewn onto the epicardial surface of a left ventricle 38. Four cooling Peltier elements 12 are provided at the center of the substrate 10, and temperature-detecting thermistors 18 are provided at part of their peripheries. Multiple circular and flat potential sensors/stimulating electrodes 14 for detecting the surface potential of the heart are arranged in a lattice form on the surface of the substrate 10 at almost equal intervals. The potential sensors/stimulating electrodes 14 are stimulating electrodes for delivering a pulse voltage/current to the heart. When ventricular tachycardia is detected, power is applied to these Peltier elements 12 to cool the heart. If the cooling cannot stop ventricular tachycardia, a pulse voltage/current is delivered via the multiple potential sensors/stimulating electrodes 14 to eliminate ventricular tachycardia at a low voltage to prevent ventricular fibrillation.

## Description

### Technical Field

The present invention generally relates to an electronic apparatus for a medical application, and more specifically, to a ventricular fibrillation preventing apparatus for preventing an occurrence of ventricular fibrillation, and to a defibrillating apparatus for stopping ventricular fibrillation.

### Background Art

There are known defibrillating apparatuses, as those disclosed in Patent Documents 1 to 6 listed below, that stop ventricular fibrillation or ventricular tachycardia upon detection thereof by applying an electrical shock to a heart by charging a capacitor and discharging a high energy electrical pulse. Such an apparatus that can be implanted into a human body is known as an implantable cardioverter defibrillator (ICD).

Patent Document 1: PCT Japanese Translation Patent Publication No. H8-509385
Patent Document 2: PCT Japanese Translation Patent Publication No. 2002-524218
Patent Document 3: PCT Japanese Translation Patent Publication No. H8-506263
Patent Document 4: PCT Japanese Translation Patent Publication No. H9-502629
Patent Document 5: Japanese Patent No. 2601762
Patent Document 6: Japanese Patent No. 3124765

### Disclosure of Invention

### Problems to be solved by the Invention

However, these defibrillators stop ventricular fibrillation and recover normal myocardial excitations by applying a strong pulse current to the entire heart within a few seconds of detection of ventricular fibrillation. Therefore, these defibrillators have the following problems. Firstly, because these defibrillators cannot prevent loss of consciousness due to ventricular fibrillation, patients subjected are_not allowed to drive a car. Secondly, upon application of the pulse voltage/current during operation of the implantable cardioverter defibrillator, the patient experiences a strong sense of discomfort and anxiety, therefore, the quality of life (QOL) of the patient is impaired. Thirdly, application of a strong current causes myocardial damage, which might result in degraded pumping function of the heart, or induction of new arrhythmias.
Furthermore, the conventional defibrillator attempts to stop ventricular fibrillation and recover normal myocardial excitations by applying a strong pulse voltage/current to the heart after detection of ventricular fibrillation. However, such defibrillators sometimes fail to stop ventricular fibrillation even when a strong current with a high voltage is repeatedly applied, and there has been a problem that application of a strong current results in degradation of pumping functions of the heart, or induction of new arrhythmias.

The present invention has been developed to solve the problems described above, and an objective of the invention is to prevent an occurrence of ventricular fibrillation. Another objective of the invention is to avoid damage to the heart as much as possible and prevent a strong sense of discomfort and anxiety, by providing a means for eliminating a spiral-formed rotating excitation (this wave, which is a distribution of cardiac potential, is referred to as a "reentrant spiral-wave" hereinafter and in the claims), which is a major mechanism of ventricular fibrillation, using a relatively weak current. Preferably, another object of the present invention is to design an apparatus with which a person would not be aware of the electrical shock application thereby. Still another objective of the present invention is, even when the apparatus operates upon the occurrence of ventricular fibrillation, to prevent heart damage as much as possible and to avoid a strong sense of discomfort and anxiety, by providing a means for controlling the applied pulse voltage/current.
Still another objective of the present invention is to promote cessation of ventricular fibrillation by providing a reversible cooling of the heart. Still another objective of the present invention is to improve the efficiency of defibrillation, by applying a pulse voltage/current of a relatively weak strength to a cooled region of the heart.

It should be understood that each of these objectives of the present invention might be achieved by any aspect of the present invention, and any aspect of the present invention might achieve only one of the above objectives; but it should not be understood that each aspect of the present invention can achieve all of the objectives simultaneously.

### Means for Solving the Problems

We discovered following facts as detailed below:
(1) By cooling a region of a heart locally, ventricular tachycardia can be stopped, and normal myocardial excitations can be recovered.
(2) When regional cooling is applied to a heart, the rotation center of reentrant spiral-waves, which are a distribution of cardiac potential, usually appears at the cooled region, and the behavior thereof becomes unstable.
(3) By cooling a region of a heart locally, a reentrant spiral-wave sometimes terminate spontaneously.
(4) By applying a pulse voltage/current to a cooled region of a heart, where the rotation center of the reentrant spiral-wave is localized, a reentrant spiral-wave can be eliminated.
(5) By applying the voltage/current at a cooled region, a ventricular reentrant spiral-wave can be stopped using a lower pulse voltage/current, compared to that required for stopping a ventricular reentrant spiral-wave without myocardial cooling.
(6) In a computer simulation using a mathematical model of heart, a critical time window for a pulse to eliminate a reentrant spiral-wave localized around the cooled region is much wider than that required for spiral-wave termination without myocardial cooling, even when a pulse of the same voltage/current is used.

The present invention has been completed based on the findings above, and is extremely unique.
A first aspect of the invention is directed to a fibrillation preventing apparatus for preventing an occurrence of ventricular fibrillation including: an electrocardiac signal detecting unit for detecting electrocardiac signals, which is an electrical potential of a heart; a cooling unit for cooling the heart; and a controlling unit for driving the cooling unit to cool the heart upon detection of a precursor phenomenon that might lead to ventricular fibrillation on the basis of the electrocardiac signals detected by the electrocardiac signal detecting unit.

The apparatus according to this aspect of the present invention operates at first to cool a heart upon detecting a precursor phenomenon that might lead to ventricular fibrillation. Myocardial cooling might terminate the precursor phenomenon, for example, ventricular tachycardia, thus preventing it from developing into ventricular fibrillation. Cooling temperature is not specified, but should be in the range that allows immediate recovery of cardiac function after stopping the cooling. For example, temperatures 3-10°C below body temperature are effective.

A second aspect of the invention is directed to a fibrillation preventing apparatus according to claim 1, wherein the precursor phenomenon is ventricular tachycardia.
Cooling of a heart is generally started at timing when ventricular tachycardia is detected. Ventricular tachycardia is the most likely cause initiate ventricular fibrillation. Because ventricular tachycardia sometimes disappears by cooling the heart, development into ventricular fibrillation can be prevented. If ventricular tachycardia disappears, it is no longer necessary to apply a pulse voltage/current, which might cause myocardial damage, to the heart.

A third aspect of the invention is directed to a fibrillation preventing apparatus according to claim 1, wherein the precursor phenomenon includes a phenomenon on the basis of an occurrence of a reentrant spiral-wave.
There is a causal relationship between ventricular tachycardia or ventricular fibrillation and a reentrant spiral-wave. An organized reentrant spiral-wave causes ventricular tachycardia. When the reentrant spiral-wave starts considerable meandering, or breaks up, the ventricular tachycardia turns into ventricular fibrillation. Therefore, this aspect of the present invention is characterized by its capability to drive the cooling unit to cool the heart upon detection of an initial occurrence of a reentrant spiral-wave.

When a regional cooling is applied to the heart, the rotation center of a reentrant spiral-wave usually appears in the cooled region, and the behavior thereof becomes unstable. Cooling alone can sometimes eliminate a reentrant spiral-wave. This can prevent development of unstable or broken reentrant spiral-waves responsible for ventricular fibrillation.

A fourth aspect of the invention is directed to a fibrillation preventing apparatus according to any one of claims 1 to 3, further including a stimulating unit for applying a pulse voltage/current to the heart, wherein the controlling unit drives the stimulating unit after a given time has elapsed since the cooling unit is driven.
By a given time elapsed since the cooling unit is driven to cool the heart, the rotation center of the reentrant spiral-wave usually arrears around the cooled region. The given time should be set to the time required for the reentrant spiral-wave to appear at the cooled region. The apparatus according to this aspect of the present invention is based on our finding that a reentrant spiral-wave can be easily eliminated by applying a pulse of a lower voltage/current if a given time has elapsed since the heart started to be cooled and the rotation center of the reentrant spiral-wave is localized in the cooled region. It is preferable that a pulse is applied close to the rotation center of a reentrant spiral-wave, that is, to an area including the cooled region. A reentrant spiral-wave can therefore be eliminated by a lower voltage/current. By driving the stimulating unit, development into ventricular fibrillation can be avoided reliably.

A fifth aspect of the invention is directed to a fibrillation preventing apparatus according to any one of claims 1 to 3, further including a stimulating unit that applies a pulse voltage/current to the heart, wherein the controlling unit detects the rotation center of the reentrant spiral-wave appearing in the cooled region of the heart after driving the cooling unit, and drives the stimulating unit on the basis of the detection. Because the rotation center of a reentrant spiral-wave shows irregular movement in the cooled region, it is also possible to detect the unstable behavior of the rotation center in the cooled region.
This aspect of the present invention is characterized in that the cooling unit and the stimulating unit are provided, and the cooling unit is driven at first, and the stimulating unit is driven to apply the pulse voltage/current at the timing when it is detected that the reentrant spiral-wave appears in the cooled region of the heart.
In other words, the apparatus according to this aspect of the present invention is based on our finding that a reentrant spiral-wave can be eliminated easily with application of a pulse having a lower voltage/current if the rotation center of the reentrant spiral-wave is located at a cooled region. In this situation, the reentrant spiral-wave can be eliminated with lower voltage/current. By driving the stimulating unit, development into ventricular fibrillation can be avoided reliably.

A sixth aspect of the invention is directed to a fibrillation preventing apparatus according to any one of claims 1 to 3, further including a stimulating unit that applies a pulse voltage/current to the heart, wherein the controlling unit drives the stimulating unit if the precursor phenomenon has not disappeared after a given time has elapsed since the cooling unit is driven.
This aspect of the present invention is characterized in that the stimulating unit is not driven if the precursor phenomenon disappears after the cooling unit is driven, because it is no longer necessary to apply a pulse voltage/current to the heart.

A seventh aspect of the invention is directed to a fibrillation preventing apparatus according to claim 2, further including a stimulating unit that applies a pulse voltage/current to the heart, wherein the controlling unit drives the stimulating unit if ventricular tachycardia has not terminated after a given time has elapsed since the cooling unit is driven.
If the ventricular tachycardia has terminated by the timing to apply the pulse voltage/current, it is no longer necessary to apply the pulse voltage/current; therefore, the pulse voltage/current is applied only if ventricular tachycardia is still detected. This prevents myocardial damage by unnecessary shocks to the heart.

An eighth aspect of the invention is directed to a fibrillation preventing apparatus according to any one of claims 4 to 7, wherein the pulse voltage/current is applied to a region being cooled by the cooling unit.
When a regional cooling is applied to the heart, the rotation center of a reentrant spiral-wave often appears in the cooled region and the behavior thereof becomes unstable. By placing stimulating electrodes in this region and applying the pulse voltage/current, ventricular tachycardia can be stopped effectively by a pulse of lower voltage/current.

A ninth aspect of the invention is directed to a fibrillation preventing apparatus according to any one of claims 4 to 8, wherein the pulse voltage/current is applied to ventricles or atria of the heart.
In the aspect of the invention according to this claim, the pulse voltage/current is applied between coil electrodes of a conventional ICD, positioned in the right ventricle and superior vena cava (or the right atrium) and a subcutaneously-implanted ICD body (can). In addition, the pulse voltage/current may be applied to a cooled region.

A tenth aspect of the invention is directed to a fibrillation preventing apparatus according to any one of claims 1 to 9, wherein the cooling unit includes Peltier elements driven by the controlling unit.
The cooling unit is not limited to the Peltier element; however, it is convenient to use the Peltier element, which converts the electric power to thermal differences and vice versa. The Peltier element is advantageous in an implantable apparatus, because it allows the size thereof to be compact.

An eleventh aspect of the invention is directed to a defibrillating apparatus including a cooling unit that provides reversible cooling of the heart.

The apparatus according to this aspect of the invention operates at first to cool the heart upon detecting ventricular fibrillation. Cooling sometimes terminates ventricular fibrillation. The cooling temperature is not specified, but should be in the range that allows immediate recovery of cardiac function after stopping the cooling. For example, temperatures 3-7°C below body temperature are effective and appropriate.

A twelfth aspect of the invention is directed to a defibrillating apparatus according to claim 11 further including a controlling unit for driving and controlling the cooling unit to cool the heart upon detecting ventricular fibrillation.
Cooling of the heart sometimes terminates ventricular fibrillation, and if ventricular fibrillation terminates by myocardial cooling alone, it is no longer necessary to apply the pulse voltage/current, which might cause myocardial damage.

A thirteenth aspect of the invention is directed to a defibrillating apparatus according to claim 11 or 12 further including an electrocardiac signal detecting unit for detecting electrocardiac signals of the heart. There are many implementations of the electrocardiac signal detecting unit, as will be described later.

A fourteenth aspect of the invention is directed to a defibrillating apparatus according to claim 13, wherein the electrocardiac signal detecting unit includes an atrial lead inserted into a cardiac atrium and a ventricular lead inserted into a cardiac ventricle.
This aspect of the present invention is characterized in that a cooling unit is additionally provided to the conventional ICD; ventricular fibrillation is detected by the conventional ICD; and the heart is cooled by the cooling unit upon detection of ventricular fibrillation.

A fifteenth aspect of the invention is directed to a defibrillating apparatus according to claim 13 further including electrocardiogram recording electrodes attached to a body surface of a patient.
This aspect of the present invention can be used for an external defibrillator. In other words, this aspect of the present invention is characterized in that a cooling unit is provided to the external defibrillator for cooling the heart. Detection of ventricular fibrillation includes a judgment by a doctor on the basis of an electrocardiogram, or automatic detection using an algorithm.

A sixteenth aspect of the invention is directed to a defibrillating apparatus according to claim 12, wherein ventricular fibrillation is detected by way of electrical signals obtained through an atrial lead inserted into a cardiac atrium and a ventricular lead inserted into a cardiac ventricle.
This aspect of the present invention is an application of the present invention to the conventional ICD, where the cooling unit is additionally provided to the conventional ICD.

A seventeenth aspect of the invention is directed to a defibrillating apparatus according to any one of claims 11 to 16 further including a stimulating unit for applying a pulse voltage/current to the heart.
This aspect of the present invention can be applied to an implantable defibrillator or an external defibrillator. The pulse voltage/current is applied at timing automatically determined by a controlling unit if it is for an implantable defibrillator, or at timing automatically determined by a controlling unit or by a doctor's judgment if it is for an external defibrillator.

An eighteenth aspect of the invention is directed to a defibrillating apparatus according to claim 17, wherein the stimulating unit is driven to apply a pulse voltage/current to the heart after the heart is cooled by the cooling unit.
Regional cooling of the heart allows the rotation center of a reentrant spiral-wave to become localized around the cooled region, and destabilized. This modification of the reentrant spiral-waves promotes their self-termination in some cases. In this manner, ventricular fibrillation can be eliminated.

A nineteenth aspect of the invention is directed to a defibrillating apparatus according to claim 17 or 18 further including stimulating electrodes for applying a pulse voltage/current to the heart region cooled by the cooling unit.
This aspect of the present invention is based on our finding that the reentrant spiral-wave can be easily eliminated with application of a reduced voltage/current if the rotation center of a reentrant spiral-wave becomes localized around the cooled region and destabilized after a given time has elapsed since the cooling of the heart is started. Preferably, the pulse voltage/current is applied to a region including an area where the reentrant spiral-wave is localized, that is, the cooled region. In this manner, a reentrant spiral wave can be eliminated and ventricular fibrillation can be stopped with a lower voltage/current.

A twentieth aspect of the invention is directed to a defibrillating apparatus according to claim 19, wherein the stimulating electrodes are provided at multiple sites on the heart surface.
A twenty-first aspect of the invention is directed to a defibrillating apparatus according to claim 17 or 18 further including stimulating electrodes positioned in the right ventricle, and the superior vena cava or the right atrium for applying a pulse voltage/current.
This aspect of the present invention is characterized in that the pulse voltage/current for termination of ventricular fibrillation is applied using coil electrodes of the conventional ICD, whose coil electrodes are positioned in the right ventricle and in the superior vena cava or the right atrium. The defibrillating apparatus having this characteristic of the present invention can be realized just by adding the cooling unit for cooling the heart to the conventional ICD. This apparatus applies a pulse voltage/current in the same manner as in the conventional ICD, therefore, has a structure of a conventional ICD equipped with the cooling unit.

A twenty-second aspect of the invention is directed to a defibrillating apparatus according to claim 17 or 18 further including stimulating electrodes attached to a body surface of a patient for applying a pulse voltage/current.
The present invention is characterized in that the pulse voltage/current is applied to stop ventricular fibrillation utilizing stimulating electrodes of the conventional external defibrillator. The defibrillating apparatus having this characteristic of the present invention can be realized just by adding the cooling unit for cooling the heart to the conventional external defibrillator.

A twenty-third aspect of the invention is directed to a defibrillating apparatus according to claim 12, wherein ventricular fibrillation is detected by electrocardiac signals obtained through electrocardiogram recording electrodes attached to a body surface of a patient.
The present invention relates to the external defibrillator, and the judgment of a fibrillation is made either automatically by the controlling unit, or by a doctor.

A twenty-fourth aspect of the invention is directed to a defibrillating apparatus according to claim 12, wherein the pulse voltage/current is applied when ventricular fibrillation has not terminated.
Because if ventricular fibrillation has terminated at the timing to apply the pulse voltage/current, it is no longer necessary to apply the pulse voltage/current; therefore, the pulse voltage/current is applied only if ventricular fibrillation is still detected. This prevents myocardial damage by unnecessary shocks.

A twenty-fifth aspect of the invention is directed to a defibrillating apparatus according to any one of claims 17 to 24, wherein the pulse voltage/current is applied to the region cooled by the cooling unit.
Regional cooling of the heart allows the rotation center of a reentrant spiral-wave to become localized around the cooled region, and destabilized. By applying a pulse voltage/current to this cooled region, the reentrant spiral-wave can be stopped effectively with a lower voltage/current.

In the above-described invention, the heart may also be cooled by driving the cooling unit at timing when ventricular tachycardia, a precursor phenomenon of ventricular fibrillation, is detected. If the phenomenon is at the stage of ventricular tachycardia, it can be eliminated more easily by cooling the heart, and the applied current or voltage can also be lowered.

### Effect of the Invention

The first, second, and third aspects of the present invention are characterized in that a cooling unit is added for cooling the heart, and the precursor phenomenon, leading to ventricular fibrillation, can be eliminated by driving the cooling unit. Ventricular tachycardia or a reentrant spiral-wave can be eliminated, and therefore, a development into ventricular fibrillation can be prevented by myocardial cooling. Furthermore, because no other interventions than cooling are applied to the heart, a burden to a patient is minimized. In addition, the patient is rarely aware of the operation of the apparatus., Implantation of the apparatus may improve QOL of the patient.

According to the forth, fifth, sixth, seventh, eighth and ninth aspects of the present invention, a pulse voltage/current is applied to a region of a heart being cooled, therefore, a precursor phenomenon, such as ventricular tachycardia or a reentrant spiral-wave, can be reliably eliminated with a low voltage, thus preventing development into ventricular fibrillation.

Cooling of the heart can prevent reentrant spiral-waves from persisting, or localize the rotation center. Under this condition, energy or pulse voltage/current required to eliminate a reentrant spiral wave or stop ventricular tachycardia may be lowered. Because a lower pulse voltage/current is applied to the heart, myocardial damage can be avoided or reduced, and a physical and mental burden to a patient can be minimized.

In addition, because the present invention can effectively eliminate a precursor phenomenon by an electrical pulse of a low voltage before ventricular fibrillation occurs, a patient is rarely aware of the apparatus being driven, therefore, QOL can be improved.
Even if ventricular fibrillation occurs, ventricular fibrillation can be stopped with a lower pulse voltage/current because the heart is being cooled. Therefore, myocardial damage can be eliminated or minimized.

The energy is applied at timing such as after a given time has elapsed since the cooling has begun and the rotation center of the reentrant spiral-wave appeared at the cooled region.

According to the sixth and seventh aspects of the present invention, if the precursor phenomenon, which can develop into ventricular fibrillation, has disappeared by the timing to drive the stimulating unit (for example, if ventricular tachycardia has stopped (claim 1), in other words, the precursor phenomenon (e.g. ventricular tachycardia (claim 7) or a reentrant spiral-wave) has been eliminated only by driving the cooling unit), the stimulating unit is not driven. Therefore, there is no burden to a patient, thus improving QOL.

The fibrillation preventing apparatus is most advantageous when it is implanted to a human body. Because the apparatus does not inhibit person's activities, thus QOL can be improved.

The eleventh and twelfth aspects of the present invention are characterized by inclusion of a cooling unit for cooling the heart, and the cardiac fibrillation can be occasionally eliminated by driving the cooling unit. In this situation, because no other interventions than cooling are applied to the heart, the burden to the patient can be reduced.

According to the thirteenth aspect of the invention, the electrocardiac signal detecting unit is provided for monitoring electrocardiac signals in order to detect an occurrence of ventricular fibrillation.
According to the fourteenth, fifteenth and sixteenth aspect of the present invention, a sensor used for detecting the electrocardiac signals is specified, allowing detection by the ICD, or the external detection.
According to the seventeenth, eighteenth, nineteenth, twentieth and twenty-first aspects of the present invention, a cooling unit is provided to cool the heart, and a controlling unit for controlling application of a pulse voltage/current to the heart; therefore, ventricular fibrillation can be stopped with a relatively low voltage/current.

Myocardial cooling can prevent a reentrant spiral-wave from persisting, or localize the rotation center thereof appears. Under this condition, energy or pulse voltage/current required to eliminate a reentrant spiral wave or stop ventricular tachycardia may be lowered. Because a lower pulse voltage/current is applied to the heart, myocardial damage can be eliminated or reduced, and physical and mental burden to a patient can be minimized.

Energy is applied at timing such as when a given time has elapsed since the cooling has started and the rotation center of a reentrant spiral-wave appears around the cooled region.
Especially, according to the nineteenth aspect of the invention, because stimulating electrodes are provided to apply a pulse voltage/current to the cooled region of the heart, ventricular fibrillation can be effectively eliminated.
According to the twentieth aspect of the present invention, because the stimulating electrodes are provided to multiple sites on the heart surface, an effective pulse voltage/current can be applied to the heart.

According to the twenty-second and twenty-third aspects of the present invention, because a pulse voltage/current can be applied and electrocardiac signals can be detected by an external defibrillator, the defibrillator according to these aspects of the present invention can be utilized as an emergency life support apparatus.
According to the fourteenth, sixteenth and twenty-first aspects of the present invention, ICDs having a defibrillator function are equipped with an additional cooling unit; therefore, ventricular fibrillation can be stopped effectively by cooling without a major modification of the apparatus.

According to the twenty-fourth aspect of the present invention, a pulse voltage/current is not applied to the heart if ventricular fibrillation has terminated by the timing to apply the voltage/current, that is, if ventricular fibrillation has terminated by driving the cooling unit alone. Therefore, a burden to a patient can be eliminated, and QOL can be improved.

According to the twenty-fifth aspect of the present invention, a pulse voltage/current is applied to the cooled region of the heart. If a pulse voltage/current is applied to the cooled region of the heart, the pulse voltage/current is applied in proximity of the rotation center of the reentrant spiral-wave; therefore, ventricular fibrillation can be stopped effectively with a relatively low voltage/current.

### Brief Description of the Drawings

Fig. 1 is a drawing for showing how fibrillation preventing apparatuses according to first to third exemplary embodiments and defibrillating apparatuses according to forth to sixth exemplary embodiments of the present invention are fitted onto a human heart;
Fig. 2 is a plan view for showing a structure of a substrate in the fibrillation preventing apparatus according to the first exemplary embodiment and that of the defibrillating apparatus according to the sixth exemplary embodiment of the present invention;
Fig. 3 is a block diagram for showing an electrical configuration of the fibrillation preventing apparatus according to the first exemplary embodiment and that of the defibrillating apparatus according to the sixth exemplary embodiment of the present invention;
Fig. 4 is a flowchart for showing process procedures of a MPU in the fibrillation preventing apparatus according to the first exemplary embodiment and the defibrillating apparatus according to the sixth exemplary embodiment of the present invention;
Fig. 5 is a waveform chart for showing a voltage waveform of a pulse voltage/current used in the fibrillation preventing apparatus according to the first to third exemplary embodiments, and defibrillating apparatuses according to the forth to sixth exemplary embodiments of the present invention;
Fig. 6 is a plan view for showing a structure of a substrate in the fibrillation preventing apparatus according to a variation of the first exemplary embodiment, and the second exemplary embodiment, and that in the defibrillating apparatus according to the sixth exemplary embodiment of the present invention;
Fig. 7 is a block diagram for showing an electrical configuration of the fibrillation preventing apparatus according to a variation of the first exemplary embodiment, and the second exemplary embodiment, and that in the defibrillating apparatus according to the sixth exemplary embodiment of the present invention;
Fig. 8 is a plan view for showing a structure of a substrate in the fibrillation preventing apparatus according to another variation of the first exemplary embodiment, and that in the defibrillating apparatus according to the forth and the sixth exemplary embodiments of the present invention;
Fig. 9 is a plan view for showing a structure of a substrate in the fibrillation preventing apparatus according to another variation of the first exemplary embodiment, and that in the defibrillating apparatus according to the fifth exemplary embodiment of the present invention;
Fig. 10 is a flowchart for showing process procedures of a MPU in the fibrillation preventing apparatus according to the second exemplary embodiment of the present invention;
Fig. 11 is a flowchart for showing process procedures of a MPU in the fibrillation preventing apparatus according to the third exemplary embodiment, and that in the defibrillating apparatus according to the forth exemplary embodiment of the present invention;
Fig. 12 is a photograph for showing a configuration of animal experiments;

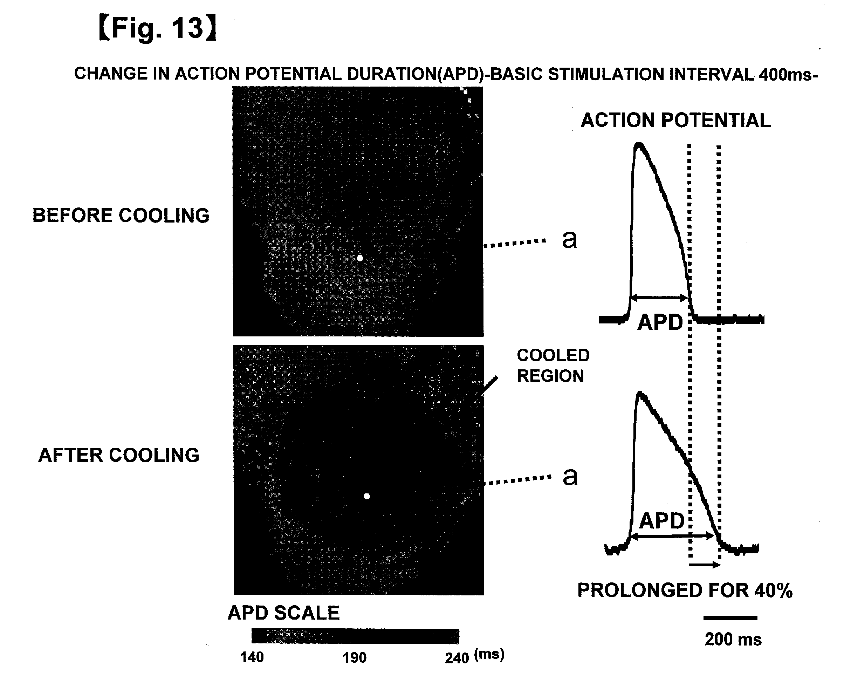

Fig. 13 is an illustration of a change in action potential configuration of the ventricle before and after regional myocardial cooling in the animal experiments;
Fig. 14 is measurement charts (isochrone activation maps during ventricular tachycardia) for showing how the rotation center of a reentrant spiral-wave (a linear trajectory thereof) is localized at a cooled region;
Fig. 15 is a phase representation of a reentrant spiral-wave before and after application of regional cooling to the heart;
Fig. 16 is an electrocardiogram chart for showing how ventricular fibrillation is stopped by regional cooling;
Fig. 17 is measurement charts for showing that a reentrant spiral-wave persists after a pulse voltage/current is applied without cooling, and that a reentrant spiral-wave is terminated by a pulse voltage/current applied after regional cooling;
Fig. 18 is measurement charts (phase maps) for showing a behavior of the rotation center of reentrant spiral-waves after regional cooling during ventricular tachycardia/fibrillation induced in a rabbit heart with myocardial infarction;
Fig. 19 is measurement charts (electrocardiograms) for showing that ventricular tachycardia/fibrillation persists after a pulse voltage/current is applied without cooling and that ventricular tachycardia/fibrillation is terminated by a pulse voltage/current applied after regional cooling;
Fig. 20 is an example of a reentrant spiral-wave dynamics during sustained ventricular tachycardia before cooling. Top, an electrocardiogram; middle, phase representation for showing a behavior of the rotation center of a reentrant spiral-wave; bottom, a graph for plotting a spatial trajectory of the rotation center over time;
Fig. 21 is a case where ventricular tachycardia is terminated by regional cooling alone. Top, an electrocardiogram; middle, a phase representation of excitation; bottom, a graph for plotting the spatial trajectory of the rotation center over time;
Fig. 22 is a graph for comparing spontaneous termination of ventricular tachycardia in 40 seconds after its initiation in the absence of cooling (Control) and presence of regional cooling;
Fig. 23 is a case where ventricular tachycardia is terminated by a combination of regional cooling and a pulse voltage/current. Top, an optical signal chart showing action potentials on the heart epicardial surface; middle, a phase representation of excitation; bottom, a graph for plotting the spatial trajectory of the rotation center over time;
Fig. 24 is a block diagram for showing an electrical configuration of the defibrillating apparatus according to the fourth exemplary embodiment of the present invention;
Fig. 25 is a diagram for showing a structure of a defibrillating apparatus according to a seventh exemplary embodiment of the present invention; and
Fig. 26 is a diagram for showing a structure of a defibrillating apparatus according to an eighth exemplary embodiment of the present invention.

### Reference Numerals

- 10: substrate
- 12, 502: Peltier elements
- 14: potential sensors/stimulating electrodes
- 16a, 16b, 402: stimulating electrodes
- 32: atrial lead
- 34: ventricular lead
- 501: stimulating paddle electrodes

### Best Mode for Carrying Out the Invention

The present invention is explained herein according to exemplary embodiments of the present invention. It should be noted that the exemplary embodiments herein are not intended to limit the scope of the present invention, and many variations thereof are still possible.

A major cause for cardiac sudden death is ventricular fibrillation, and most ventricular fibrillations are triggered by ventricular tachycardia. In recent years, great technological improvements in optical mapping have come to allow observation of complex cardiac excitations as fluorescent signals, and it has been demonstrated that a reentrant spiral-wave plays an important role in the occurrence of ventricular tachycardia or ventricular fibrillation.

We attempted to eliminate a reentrant spiral-wave by applying moderate regional cooling (3-10°C) in order to localize the rotation center (core region) of the reentrant spiral-wave as well as to destabilize the rotation thereof, and by effectively applying weak electric stimulation in proximity to the core region, where the myocardium is most easily electrically captured.

We have confirmed in animal experiments that a reentrant spiral-wave can be terminated by application of a much lower pulse voltage/current, if it is applied subsequent to regional cooling of the heart, compared with the pulse voltage/current required for termination of ventricular fibrillation without regional cooling.
Results of the animal experiments have also been theoretically supported by a computer simulation using mathematical models of the heart, which demonstrated a causal relationship between regional cooling of a heart and lowering of a voltage/current used for electrical defibrillation.

The animal experiments were conducted in the manner described below.
A heart was excised from a rabbit, and the coronary arteries thereof were perfused. Tissues from the endocardial myocardium of the left ventricle were cryoablated, and only the myocardial layer under the epicardium (approximately 1mm in thickness) was left out to create a two-dimensional perfused ventricular myocardium. This preparation allow us to analyse the detailed dynamics of two dimensional spiral waves.

In some of the animal experiments, microbeads were injected into the coronary arteries of rabbits to cause nontransmural myocardial infarction, and the rabbits two weeks after the operation were used for the experiments. The endocardial and midcardia layers of the left ventricles were not cryoablated in the heart with myocardial infarction. The heart was stained using a voltage-sensitive dye (Di-4-ANEPPS), and images of potential fluorescent signals on the epicardial surface of the anterior left ventricle were captured using a high-speed video camera. The myocardial temperature of a part of the left ventricle was decreased by 3-7°C using a cooling probe (8-10mm in diameter) or a cool-water perfusion apparatus (10mm in diameter) incorporated into a transparent acrylic board.

Electrical stimuli (basic stimulation) at 2.5 Hz (400ms intervals) were applied from a cardiac apex, and action potential duration (APD) and conduction velocity in the ventricular myocardium were measured. During a vulnerable period of action potentials in the basic stimulation, extra stimuli were applied to the heart from electrodes placed both sides of the right and left ventricles, to induce ventricular tachycardia resulting from a reentrant spiral-wave. Then, biphasic direct current was applied from unipolar or bipolar electrodes in an attempt to stop the reentrant spiral-wave.

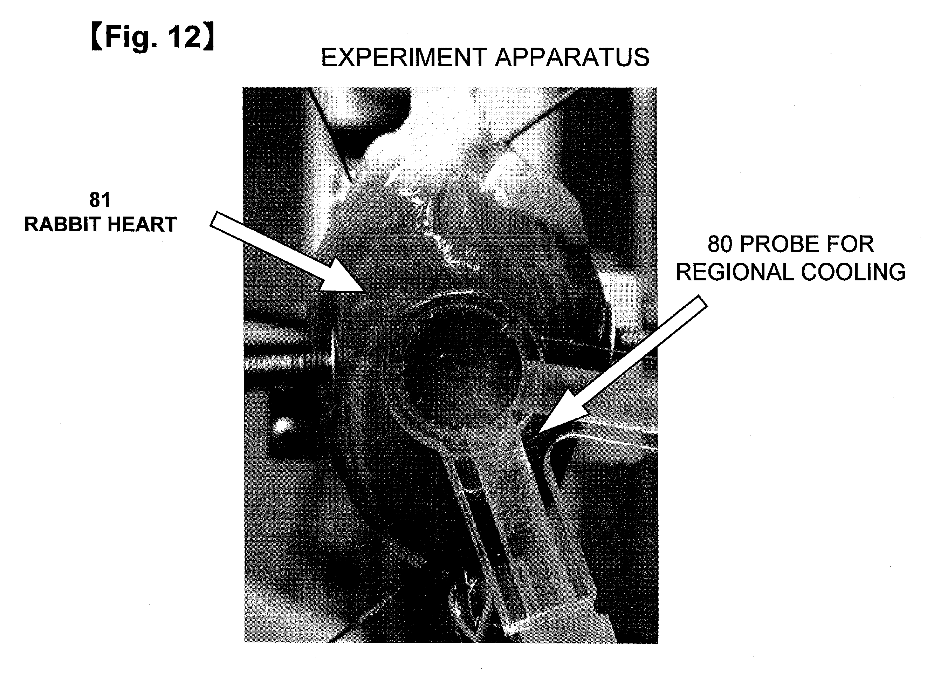

We conducted pilot experiments by placing a cooling probe (cool-water perfusing unit) 80 shown in Fig. 12 onto a rabbit heart 81 (a two-dimensional perfused heart with left ventricular endo-midmyocardial cryoablatation of a normal rabbit). In this experiment, when regional cooling (with 3-7°C lower temperature) was applied to a circular region of the anterior left ventricle, the action potential duration (APD) of the cooled region was prolonged by 30-40%, and this change was reversible. Conduction velocity decreased moderately.

When ventricular tachycardia was induced by applying the electrical stimuli to the two-dimensionally perfused heart of normal rabbits, a reentrant spiral-wave was observed in the anterior left ventricular surface in eight cases out of twenty one. (In the remaining thirteen cases, a unidirectional excitation wave was observed, and we concluded that the center of the reentrant spiral-wave was located outside of the observed area.) The rotation center of the reentrant spiral-wave often showed small meandering along a linear trajectory. When only a single stable reentrant spiral-wave existed, electrocardigrams showed ventricular tachycardia, and when the spiral-wave exhibited break-up or large irregular meandering electrocardiogram turned into ventricular fibrillation (precursor phenomenon).

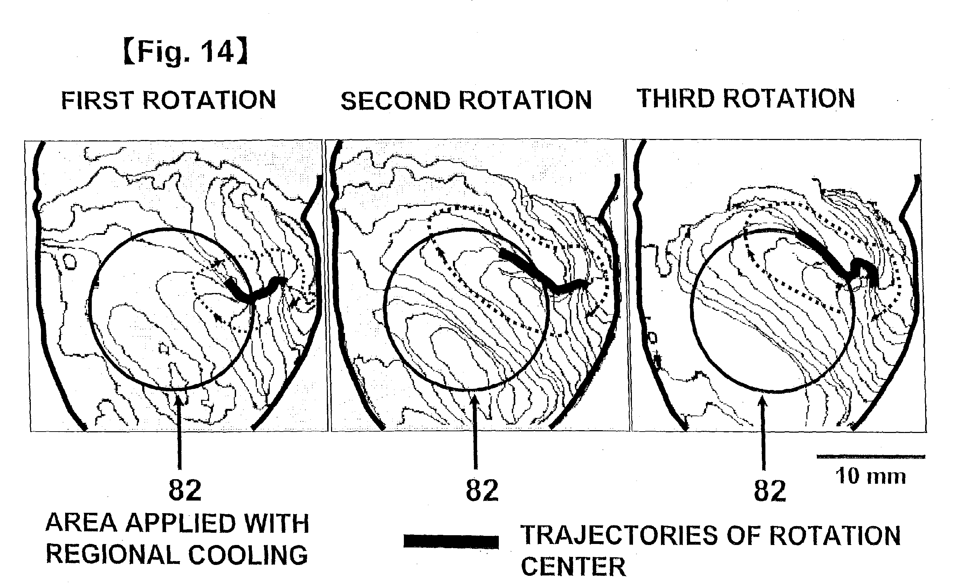

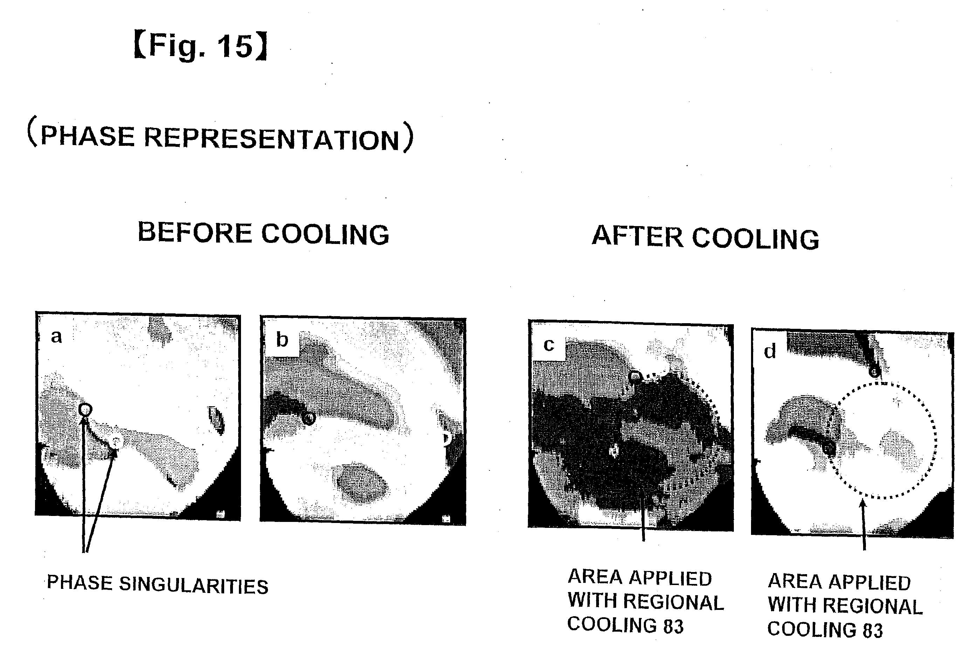

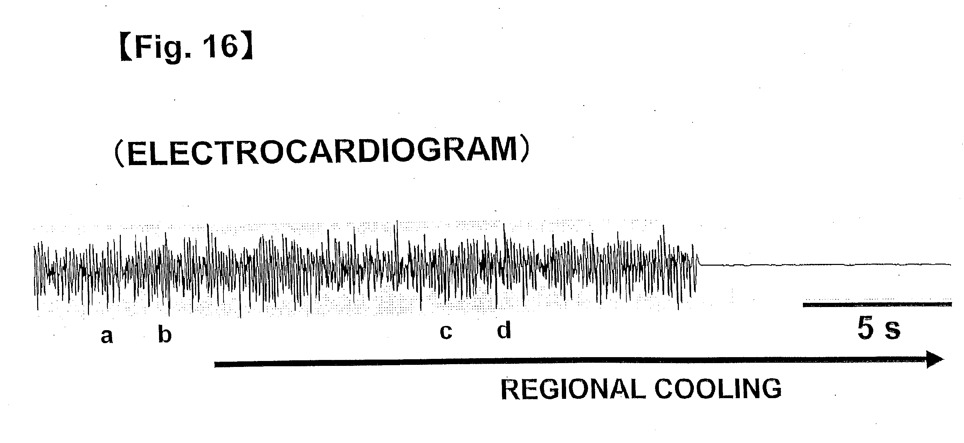

On the other hand, if a reentrant spiral-wave was induced after application of the regional cooling to the anterior left cardiac ventricle with the cooling probe 80, the rotation center thereof always localized around the cooled region 82 as shown in Figs. 14 and 15. Also, seventeen out of twenty reentrant spiral-waves stopped as shown in Fig. 16. These results indicate that the movement of reentrant spiral-waves can be controlled by application of regional cooling to a part of the heart, and application of regional cooling has an effect to promote their self-termination.

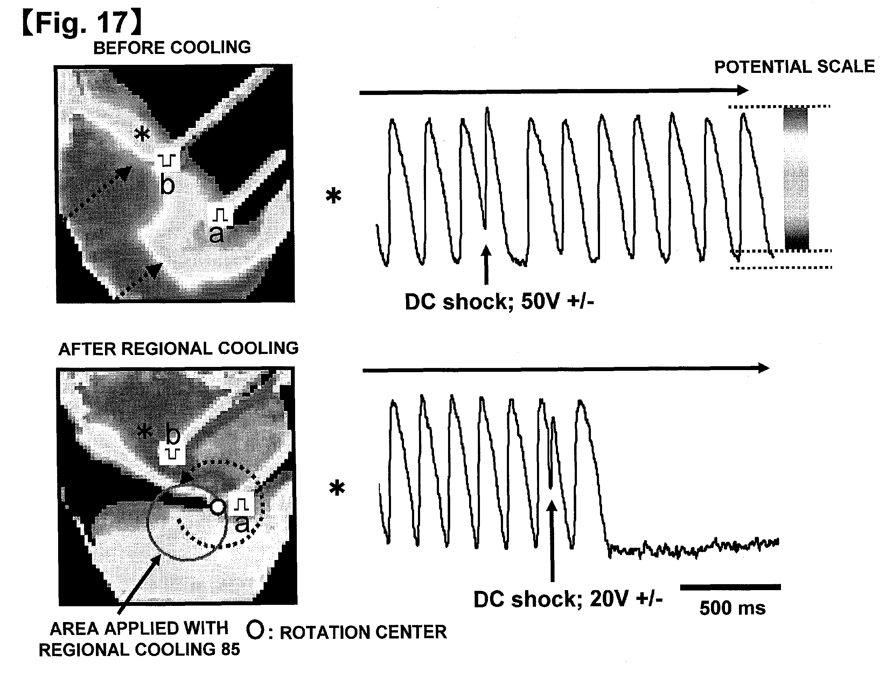

Furthermore, by applying the regional cooling, the reentrant spiral wave was stopped in four cases out of eleven. Also in the remaining seven cases, the reentrant spiral wave was stopped in four cases out of seven by applying a direct current of a relatively low voltage at the sites "a" and "b", as shown in Fig. 17, in proximity of the cooled region 85. These results indicate that regional cooling promotes cessation of a reentrant spiral-wave induced by application of electrical stimuli, and this is the first ever report of such a finding.

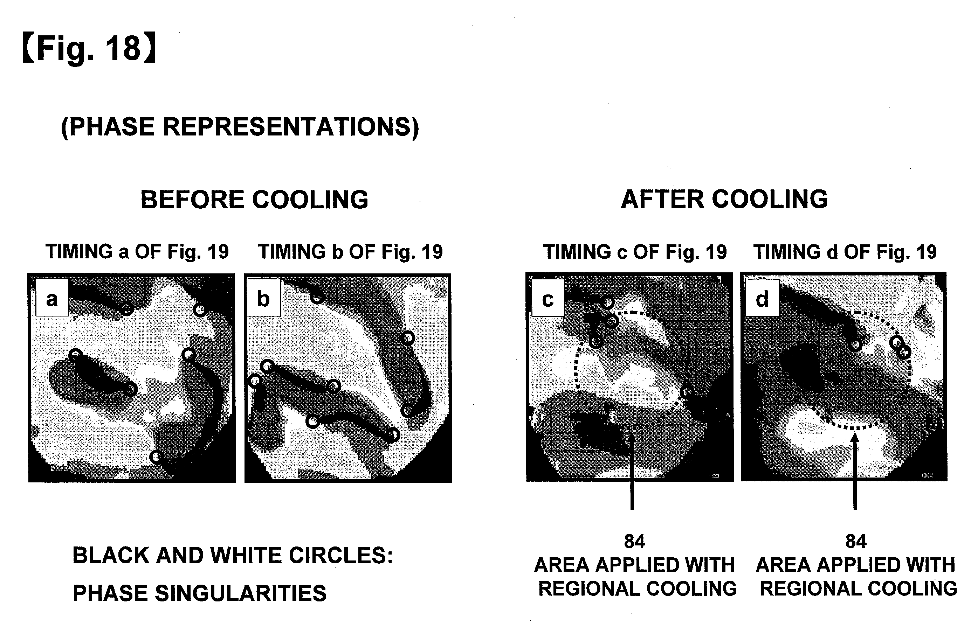

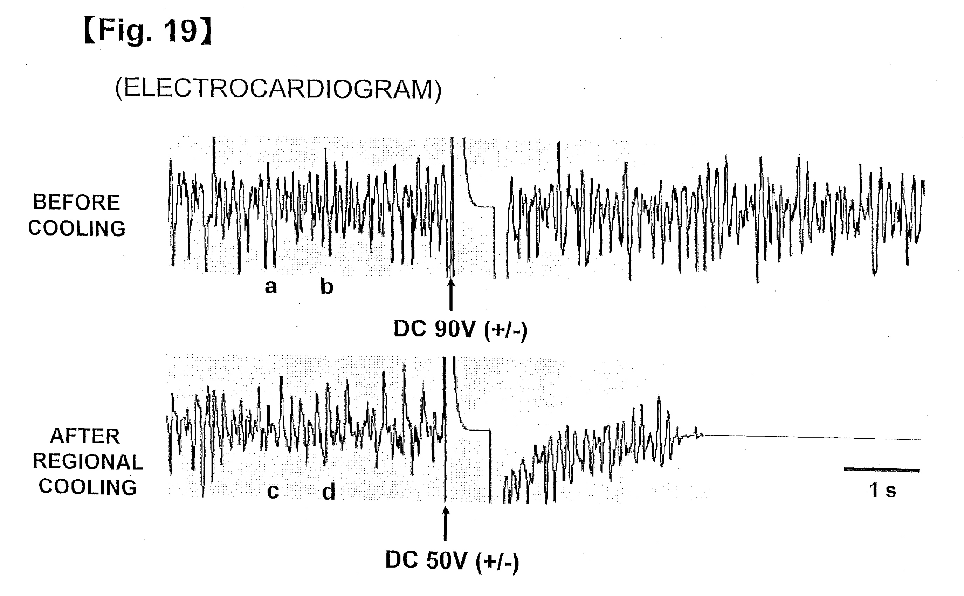

In the experiments using rabbits with nontransmural myocardial infarction, ventricular fibrillation was induced by applying electrical stimuli (DC shock). In the fluorescence images of action potential signals on the left ventricular surface during ventricular fibrillation, multiple phase singularities (corresponding to the centers of the reentrant spiral-waves) always existed as shown in a measurement chart before cooling in Fig. 18, and their locations were constantly changing. This indicates frequent and complex breakup of reentrant spiral-waves underlying the ventricular fibrillation.

When regional cooling (of 10mm diameter) was applied by the cooling probe to a part of the left ventricle, the phase singularities were localized around peripheries of the cooled region 84 in eight cases out of eight, and ventricular fibrillations stopped in two cases. When a direct current was applied with a relatively low voltage in proximity to the cooled region, ventricular fibrillation stopped in three cases out of six, as shown in the electrocardiogram of Fig. 19. The voltage of the direct current (defibrillation threshold) required to stop the fibrillation decreased by approximately 50% after the regional cooling. This indicates that application of regional cooling can reduce the energy (voltage x current) of an electrical stimulus (pulse) required to stop ventricular fibrillation.

From the animal experiments described above, we confirmed that, by cooling the heart, the rotation center of the reentrant spiral-wave are localized in the cooled region and the rotations thereof become destabilized, and the reentrant spiral-wave can be eliminated by applying a lower voltage/current, allowing ventricular tachycardia and ventricular fibrillation to stop, and normal cardiac excitations to recover. We also confirmed that, in some cases, the reentrant spiral-waves self-terminated just by applying regional cooling to the heart, and, therefore, ventricular tachycardia and ventricular fibrillation can be stopped.

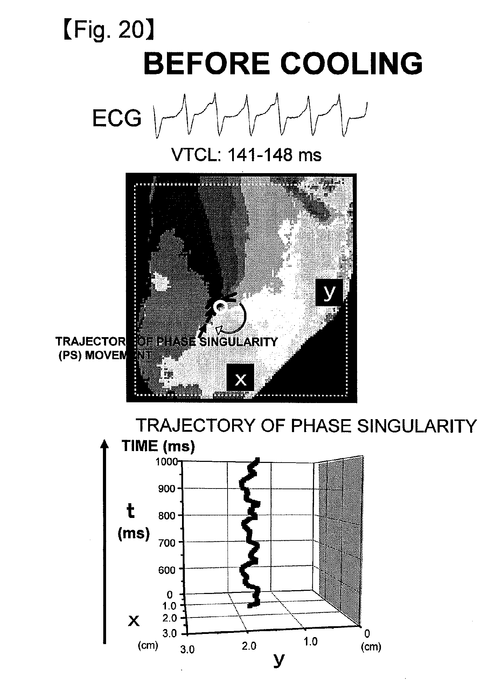

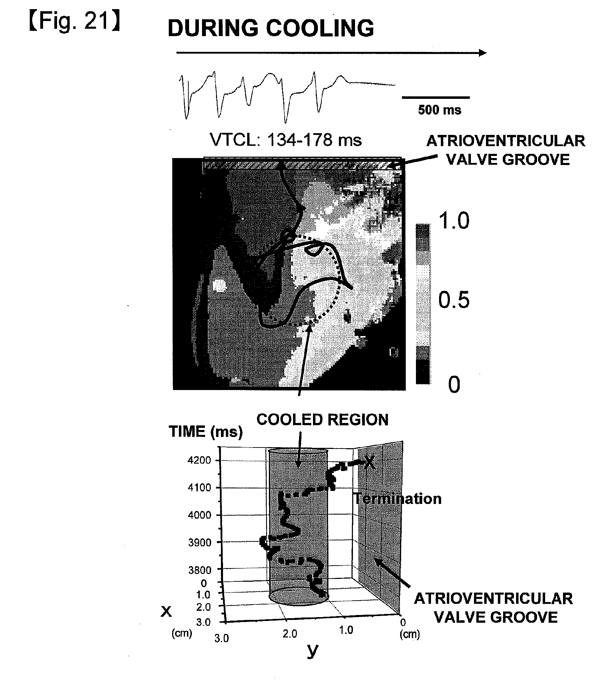

The above experiments were repeated, and spatial behaviors of the reentrant spiral-waves over time were analyzed in detail. Behaviors of the rotation centers of the reentrant spiral-wave are shown in Figs. 20 and 21. In the experiment example shown in Fig. 20, the reentrant spiral-wave was located mostly at the center of the anterior left ventricle, and the rotation thereof was relatively stable, although small meandering was observed. When regional cooling was applied under this condition, the rotations of the spiral-wave became destabilized, and the rotation center of the reentrant spiral-wave showed irregular meandering along the edge of the cooled region on the heart surface. Finally, the rotation center was detached from the edge of the cooled region, and collided with the atrioventricular valve groove, which is a non-excitable boundary of the ventricle, and the reentrant spiral-wave disappeared.

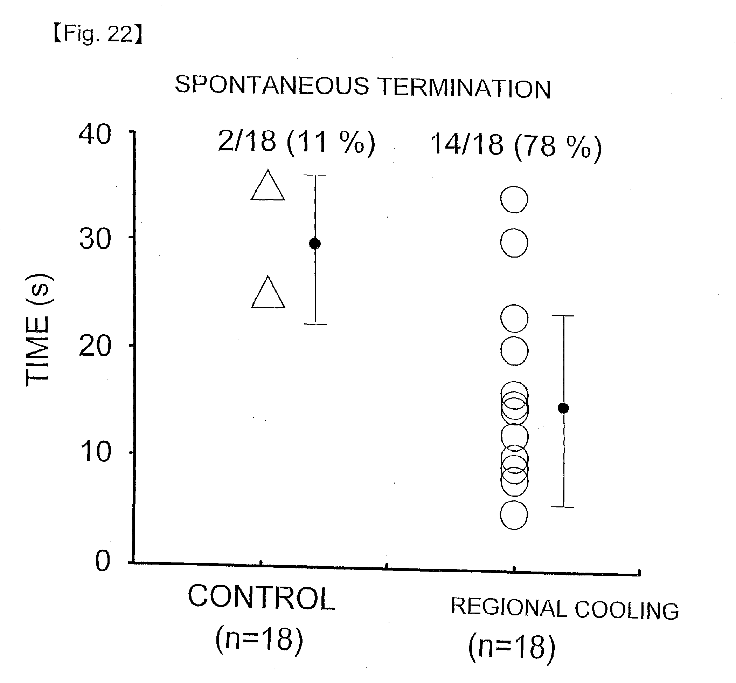

Fig. 22 shows the probability of spontaneous termination of sustained ventricular tachycardia within 40 seconds. Sustained ventricular tachycardia (>120 seconds) were induced by an electrical shock. In control (before cooling), in two out of fourteen cases (11%) reentrant spiral-wave terminated spontaneously at 30 seconds in average. When regional cooling (by 3-7°C) was applied, in contrast, in fourteen out of eighteen(78%), reentrant spiral-waves terminated at 15 seconds in average.

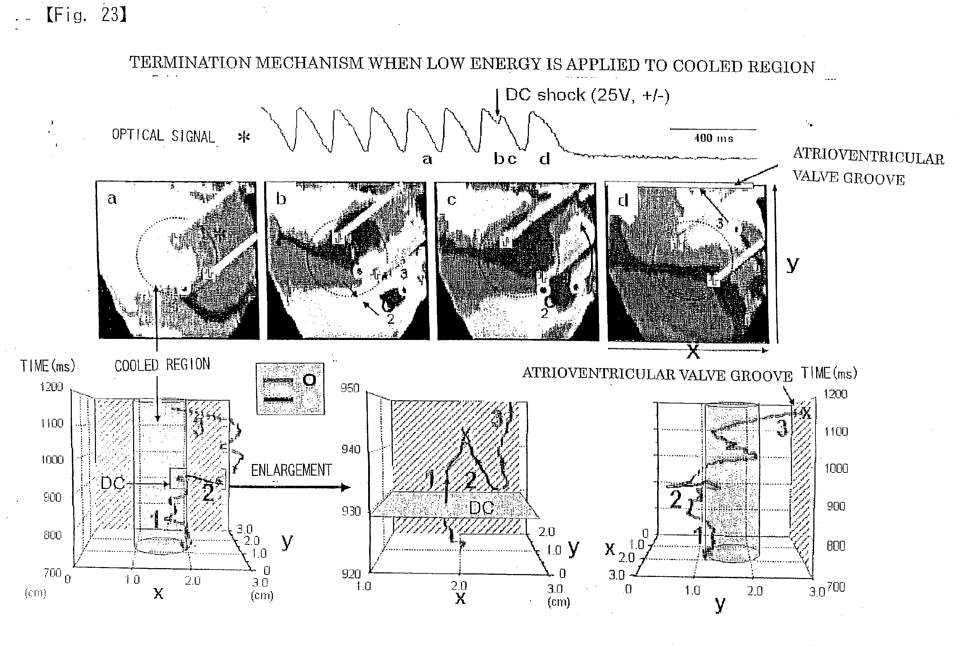

Subsequently, a relatively weak pulse voltage/current (DC 25V, biphasic) was applied in cases in which a reentrant spiral-waves did not terminate after regional cooling. The results are shown in Fig. 23. In the phase representations of excitation shown in the middle row in Fig. 23, "a" indicates a state of the reentrant spiral-wave before the pulse voltage/current was applied; "b" indicates a state of the reentrant spiral-wave during application of the pulse voltage/current; and "c" and "d" indicate states of the reentrant spiral-wave after the pulse voltage/current was applied. The figures in the lowest row show the trajectory of the rotation center of the reentrant spiral-wave when the pulse voltage/current was applied (plotted with respect to space and time). White and black circles indicate phase singularities (rotation center) (the white circles indicate clockwise rotation, and the black circles indicate counterclockwise rotation). Before the pulse voltage/current was applied, only one reentrant spiral-wave was present (phase singularity 1), as shown in "a". As shown in "b", it can be observed that two new phase singularities 2 and 3 were formed upon application of the pulse voltage/current. Then, the reentrant spiral-waves 1 and 2 collided with each other (mutual annihilation) and disappeared, and the other reentrant spiral-wave remained and kept rotating. Subsequently, the remaining reentrant spiral-wave (phase singularity 3) showed large meandering and finally collided with the atrioventricular groove and disappeared, giving rise to its disappearance.

On the basis of these observations, we concluded that:
(1) regional cooling of the myocardium localizes the rotation center of a reentrant spiral-wave around the cooled region and destabilizes the rotation thereof, promoting termination of the reentrant spiral-wave; and
(2) termination of the reentrant spiral-wave by regional cooling is facilitated by application of a weak pulse current/voltage.
These are our findings.
The present invention is novel and a unique fibrillation preventing apparatus designed on the basis of these experimental findings.

### EMBODIMENT 1

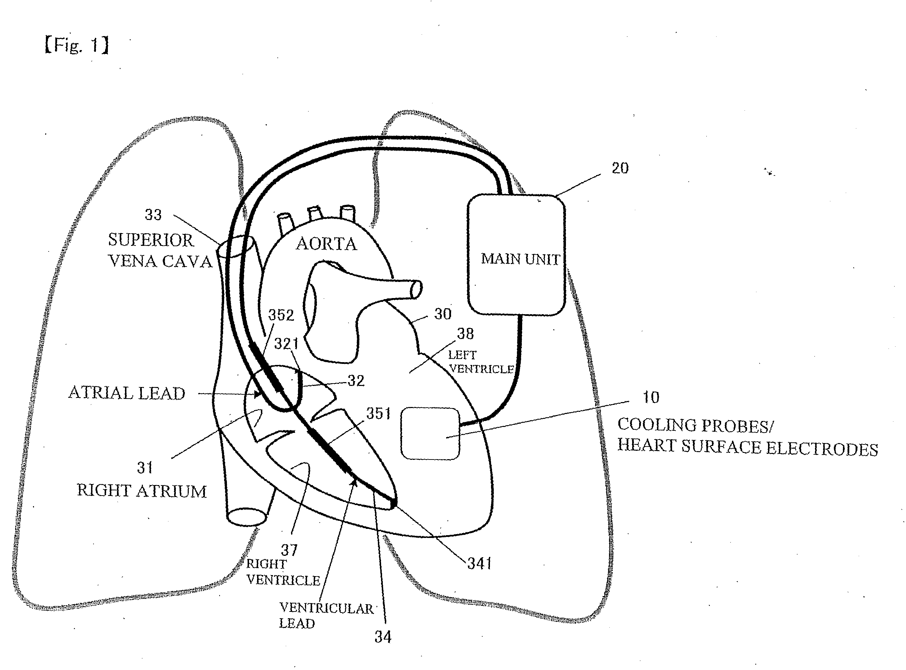

Fig. 1 shows how a fibrillation preventing apparatuses according to a first exemplary embodiment of the present invention is mounted on a heart 30.
An atrial lead 32 is inserted through the superior vena cava 33 into the right atrium 31. An electrode 321 is attached to a tip of the atrial lead 32 to detect myocardial potentials of the right atrium 31. In a similar manner, a ventricular lead 34 is inserted through the superior vena cava 33 into the right ventricle 37. An electrode 341 is attached to a tip of the ventricular lead 34 to detect myocardial potentials of the right ventricle 37. Stimulating coil electrodes 351, 352 are provided on the ventricular lead 34 in the positions of the right ventricle and superior vena cava (or the right atrium). It should be noted that these lead electrodes may be placed in any positions on the heart. The myocardial potential may be detected at any suitable position depending on the types of heart state information to be collected or controlling methods. For example, the leads may be provided so that the electrodes are placed in the left atrium or the left ventricle.

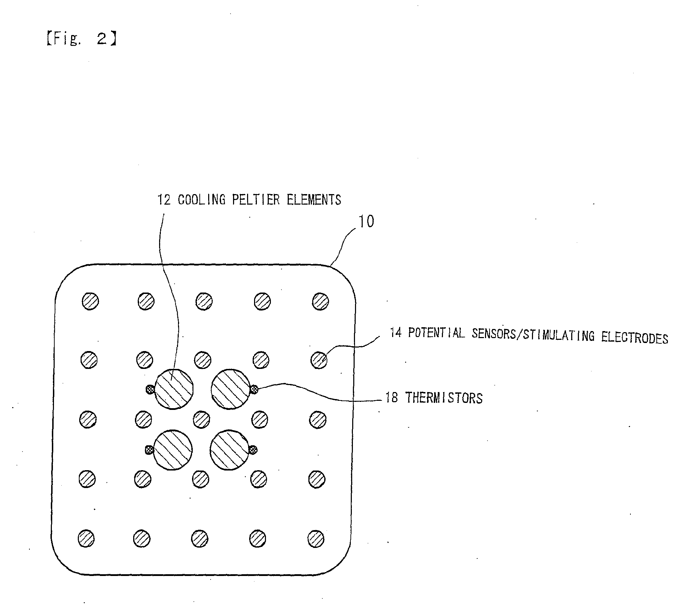

A flexible rectangular substrate 10, which is a characterizing aspect of the present invention, is attached onto the epicardium of the left ventricle 38. As shown in Fig. 2, four cooling Peltier elements 12 (cooling units) are provided at the center of the substrate 10. Each Peltier element 12 has a thermistor 18 (temperature detecting unit) at a point on the circumference thereof to detect temperature. On the surface of the substrate 10, multiple potential sensors/stimulating electrodes 14, each having a form of a circular flat plate, is arranged in a lattice at almost equal intervals to detect the potential on the heart surface. These potential sensors/stimulating electrodes 14 also function as stimulating electrodes that apply a pulse voltage/current to the heart. The Peltier elements 12, the thermistors 18 and the potential sensors/stimulating electrodes 14 are fixed in close contact with the epicardium of the left cardiac ventricle.

A main unit 20 having a controlling unit is implanted subcutaneously in the precordial region of a person. The controlling unit is input with signals from the potential sensors/stimulating electrodes 14 on the substrate 10 and electrocardiac signals input from the atrial lead 32 and the ventricular lead 34, and controls the pulse voltage/current to be output via these electrodes and leads.

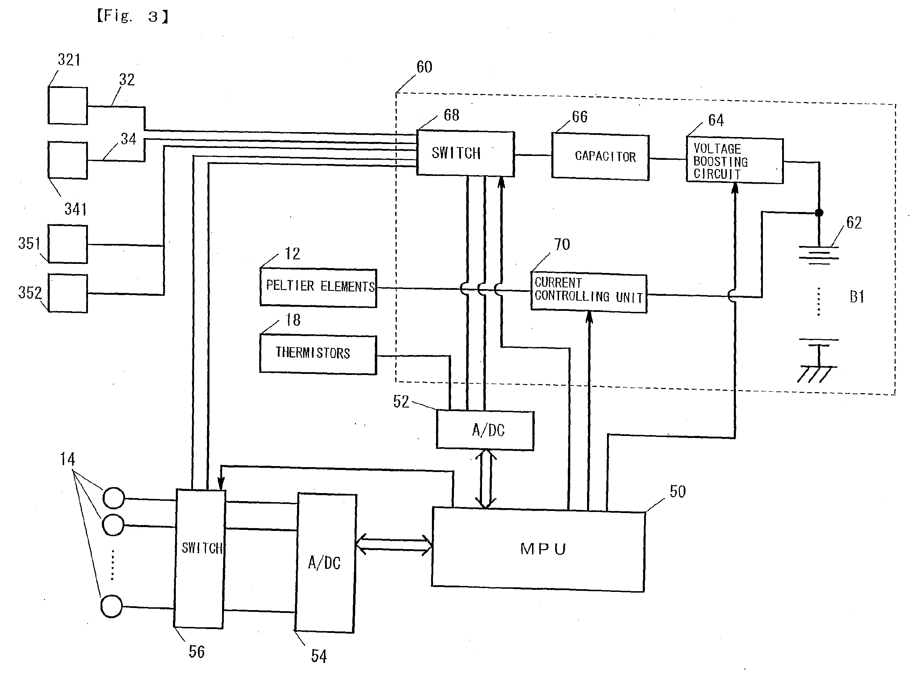

Fig. 3 shows an electrical configuration of the fibrillation preventing apparatus according to the exemplary embodiment of the present invention. The controlling unit mainly includes an MPU (microprocessor unit) 50 for detecting the electrocardiac signals, processing the detected signals and controlling application of the power, and A/D converters 52, 54. The electrocardiac signals from the potential sensors/stimulating electrodes 14 are input to the A/D converter 54 via a switch 56, converted into digital signals, and read and processed by the MPU 50.

A stimulating unit 60 mainly includes a battery 62, a voltage boosting circuit 64 for increasing the voltage input from the battery 62 on the basis of an instruction received from the MPU 50, a capacitor 66 for storing the electric charge by the voltage increased by the voltage boosting circuit 64, a switch 68 for controlling the conductivity of input and output terminals and the switching thereof, and a current controlling unit 70 for controlling the conduction of a current to the Peltier elements 12 on the basis of instructions received from the MPU 50. The controlling unit and the stimulating unit described above are incorporated into the main unit 20 shown in Fig. 1.

The switch 68 controls switching of a transmission system in a circuit described below. Upon receiving the instruction from the MPU 50, the switch 68 is switched to output the electrocardiac signals, received from the electrode 321 attached to the tip of the atrial lead 32 and the electrode 341 attached the tip of ventricular lead 34, to the A/D converter 52. The switch 68 is also switched according to an instruction from the MPU 50, so that the voltage from the capacitor 66 is output to the coil electrodes 351, 352.

The switch 56 is also switched according an instruction from the MPU 50 to output the voltage in the capacitor 66 to the potential sensor/stimulating electrodes 14 arranged on the substrate 10.

Signals output from the thermistors 18 are read into the MPU 50 via the A/D converter 52. The current controlling unit 70 operates under the control of the MPU 50 to supply electricity to the Peltier elements 12, changing the cooling capability thereof; in this manner, heart temperature is controlled to bring the temperature detected by the thermistors 18 to a target temperature.

The electrocardiac signal detecting unit according to the exemplary embodiment of the present invention mainly includes the atrial lead 32, the electrode 321 located at the tip thereof, the ventricle lead 34, the electrode 341 located at the tip thereof, and the potential sensors/stimulating electrodes 14. The controlling unit mainly includes the MPU 50, and the A/D converters 52, 54 and the thermistors 18. The cooling unit includes the Peltier elements 12. The stimulating unit includes the battery 62, the voltage boosting circuit 64, the capacitor 66, the switches 68, 56, the potential sensors/stimulating electrodes 14 and the current controlling unit 70.

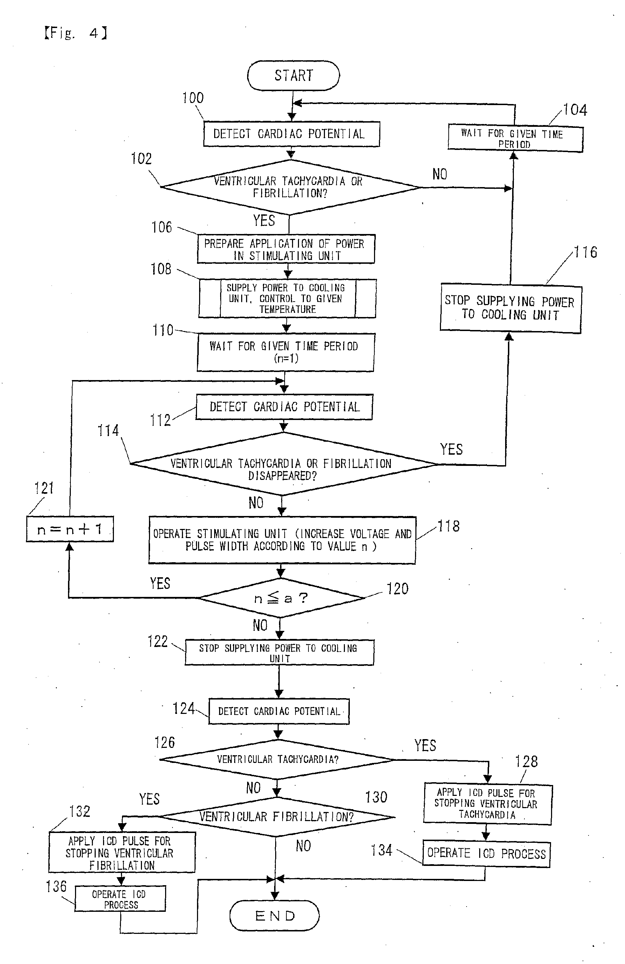

Operation of the fibrillation preventing apparatus according to the exemplary embodiment is explained below with reference to Fig. 4 showing the operation as procedures executed by the MPU 50.
At a step 100, the switch 68 is switched so as to be input with the signals from the atrial lead 32 and the ventricular lead 34. In this manner, the potentials at the myocardiums in the right atrium 31 and the right ventricle 37 are detected. The switch 56 is switched so as to be input with the potential signals from the potential sensors/stimulating electrodes 14, and by receiving these potential signals, the potential distribution on the epicardium of the left ventricle is detected. These signals are electrocardiac signals representing myocardial excitations. At a step 102, these electrocardiac signals are analyzed to determine whether ventricular tachycardia or ventricular fibrillation has occurred or not. It is determined whether the signals indicate ventricular tachycardia or not on the basis of the excitation cycle (ventricular rate) and duration thereof. It is important not to misconceive supraventricular arrhythmia, such as sinus tachycardia or atrial fibrillation, as ventricular fibrillation.

Here, ventricular tachycardia is detected as a precursor phenomenon that is likely to develop into ventricular fibrillation. The presence of ventricular tachycardia is determined on the basis of the cycles and duration of the electrocardiac signals received from the electrode 341, located at the tip of the right ventricle lead 34, and the potential sensors/stimulating electrodes 14. A reentrant spiral-wave can be detected on the basis of a time and space distribution of the electrocardiac signals received from the potential sensors/stimulating electrodes 14. The presence of supraventricular arrhythmias, such as sinus tachycardia, atrial fibrillation or flutter, is determined on the basis of the electrocardiac signals received from the electrode 321 located at the tip of the right atrial lead 32. If such supraventricular arrhythmias are detected, the apparatus is not driven. If ventricular fibrillation is detected, the apparatus is driven. Ventricular tachycardia or ventricular fibrillation can be detected by using an arrhythmia-detecting algorithm used in a known ICD (implantable cardioverter defibrillator).

If ventricular tachycardia or ventricular fibrillation is not detected, the apparatus is not driven; and the system control proceeds to a step 104, and returns to the step 100 after a given wait time. The electrocardiac signals are continuously monitored by looping the steps 100-102-104.

If ventricular tachycardia or ventricular fibrillation is detected at the step 102, a control signal is output to the voltage boosting circuit 64, and the capacitor 66 is charged to a given voltage. In other words, preparation for application of the pulse voltage/current to the heart takes place in advance. The voltage/current used here is lower than that used in the known ICD.

Subsequently, the system control proceeds to a step 108, where power is supplied to the Peltier elements 12, which constitute a cooling unit, and a control signal is output to the current controlling unit 70 to reduce the regional temperature of the left ventricular epicardium from the body temperature by 5°C. Upon doing so, temperature detection signals from the thermistors 18 are input, and the power supplied from the current controlling unit 70 is controlled by feedback thereby to bring the detected temperatures to the target temperature. By way of this control, the regional temperature of the left ventricular epicardium 38 reaches the specified target.

At the step 110, after the system control has waited until the epicardial temperature reaches the specified temperature, a voltage boosting parameter n is set to 1 initially, and the system control proceeds to a step 112, at which point the cardiac potential is detected by each sensor, in the exact same manner as at the step 100. Subsequently, the system control proceeds to a step 114 where it is determined whether ventricular tachycardia or ventricular fibrillation has terminated; if it has terminated, the system control proceeds to a step 116 where the power supply to the Peltier elements 12 is stopped. In the manner described above, the system control of the MPU 50 returns to the step 100, and the heart monitoring loop including the steps 100-102-104 is executed.

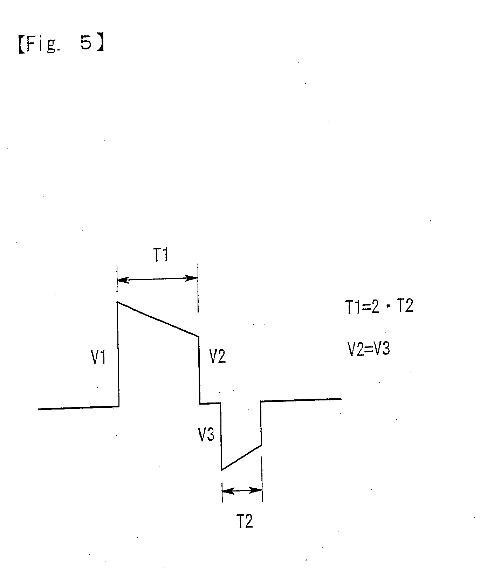

At the step 114, if it is determined that ventricular tachycardia or ventricular fibrillation has not terminated, the system control proceeds to a step 118. At the step 118, the potential sensors/stimulating electrodes 14 are connected to the stimulating unit 60, and the switch 56 is switched so as to enable application of the pulse voltage/current. Then, the stimulating unit 60 is operated to apply the pulse voltage/current to the potential sensors/stimulating electrodes 14. Voltage of a biphasic waveform, as shown in Fig. 5, is used as a voltage of the pulse voltage/current. At this time, voltage V1 and pulse width T1 are modified in proportion to the voltage boosting parameter n. The voltage/current is applied between two groups of the potential sensors/stimulating electrodes 14 that are grouped so as to sandwich the cooled region.

In the manner described above, the pulse voltage/current is applied between the potential sensors/stimulating electrodes 14 at the step 118, and the pulse current flows into the cooled region in the left ventricle. At a step 120, if a control repetition parameter n is equal or below a maximum value a, the repetition parameter n is incremented by 1 at a step 121. Then, the system control returns to the step 112 to repeat a loop, to detect the cardiac potential and to determine whether ventricular tachycardia or ventricular fibrillation has terminated.

If ventricular tachycardia or ventricular fibrillation has not terminated, the voltage of the pulse voltage/current is boosted for a given amount and the pulse width thereof is increased so as to enhance the applied energy, and then the energy is applied. This is repeated until ventricular tachycardia or ventricular fibrillation terminates, or until the number of repetitions exceeds the maximum number of repetitions a. If ventricular tachycardia or ventricular fibrillation has not terminated and the number of repetitions exceeds the maximum number of repetitions a, application of power to the Peltier elements 12, which is the cooling units, is stopped at a step 122. At a step 124, the cardiac potential is detected again by each of the sensors 14, the atrial lead 32 and the ventricular lead 34.

Subsequently at a step 126, if ventricular tachycardia is detected, the system control proceeds to a step 128, and a pulse voltage/current used for removing ventricular tachycardia in the known ICD is applied. The pulse voltage/current is applied between the coil electrodes 351, 352 of the ventricular lead 34 which is positioned in the right ventricle and the superior vena cava (or right atrium), and the package case (can) of ICD main unit. In other words, if ventricular tachycardia cannot be eliminated using the method that applies a pulse voltage/current to the cooled region, the voltage/current is applied between the ventricle and the case of ICD main unit. Subsequently, processes of the known ICD take place at a step 134, and the system control of the apparatus ends when control ending conditions of the known ICD are established. Then, the system control is started again from the step 100.

If it is determined that ventricular fibrillation cannot be terminated at a step 130, the system control proceeds to a step 132, and a pulse voltage/current used for termination of ventricular fibrillation in the known ICD is applied. The pulse voltage/current is applied between the coil electrodes 351, 352 of the ventricular lead 34, positioned in the right ventricle and the superior vena cava (or right atrium), and the ICD main unit. At this time, a high pulse voltage/current is applied. Subsequently, processes of the known ICD take place at a step 136, and the control of the apparatus ends when control ending conditions of the known ICD are met. Then, the system control is started again from the step 100.

In the above exemplary embodiment of the present invention, the pulse voltage/current is applied between the two groups of the potential sensors/stimulating electrodes 14 at the step 118; however, the pulse voltage/current may also be applied between the coil electrodes 351, 352 attached to the ventricular lead 34 and the potential sensors/stimulating electrodes 14. Alternatively, combination thereof may be used. In other words, the pulse voltage/current may be applied between two groups of the potential sensors/stimulating electrodes 14, and between one of the two groups of the potential sensors/stimulating electrodes 14 and the coil electrodes 351, 352 of the ventricular lead 34 positioned in the right ventricle and the superior vena cava (or right atrium). Furthermore, it is also possible to apply the pulse voltage/current between the potential sensors/stimulating electrodes 14 and the case of main unit 20.

Also in the above exemplary embodiment, a precursor phenomenon that is likely to develop into ventricular fibrillation is ventricular tachycardia, and cooling of the heart is started when ventricular tachycardia is detected; however, other precursor phenomena leading to ventricular fibrillation may also used as the condition to start cooling. Also, if it is possible to create a program to detect a reentrant spiral-wave by measuring the potential distribution of the entire heart, the cooling may also be started when the reentrant spiral-wave is detected. Furthermore, application of the pulse voltage/current is started when a given time has elapsed after the cooling of the heart was started; however, it is also possible to detect localization of the rotation center of the spiral wave at the cooled region using the potential sensors/stimulating electrodes 14, and to apply the pulse voltage/current when the reentrant spiral-wave becomes localized at the cooled region.

The voltage and the pulse width of the pulse voltage/current is changed according to the voltage boosting parameter n, which is the repetition parameter; however, application of the pulse voltage/current may be stopped after being carried out once. Alternatively, it is also possible to gradually increase the voltage and the pulse width according to the voltage boosting parameter n when ventricular tachycardia is detected, and to apply a pulse voltage/current of a given high voltage only one time when ventricular fibrillation is detected. According to the above exemplary embodiment of the present invention, the main unit 20 of the fibrillation preventing apparatus is implanted subcutaneously in the precordial region of a person; however, it may also be controlled from outside of a person, without implanting.

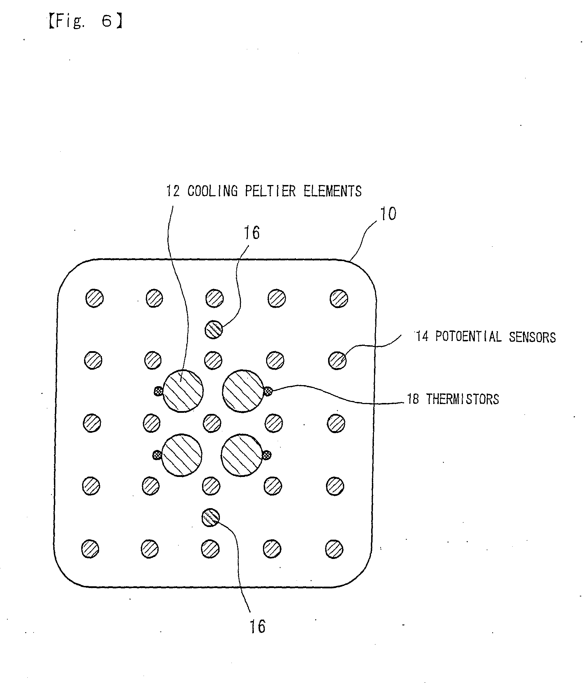

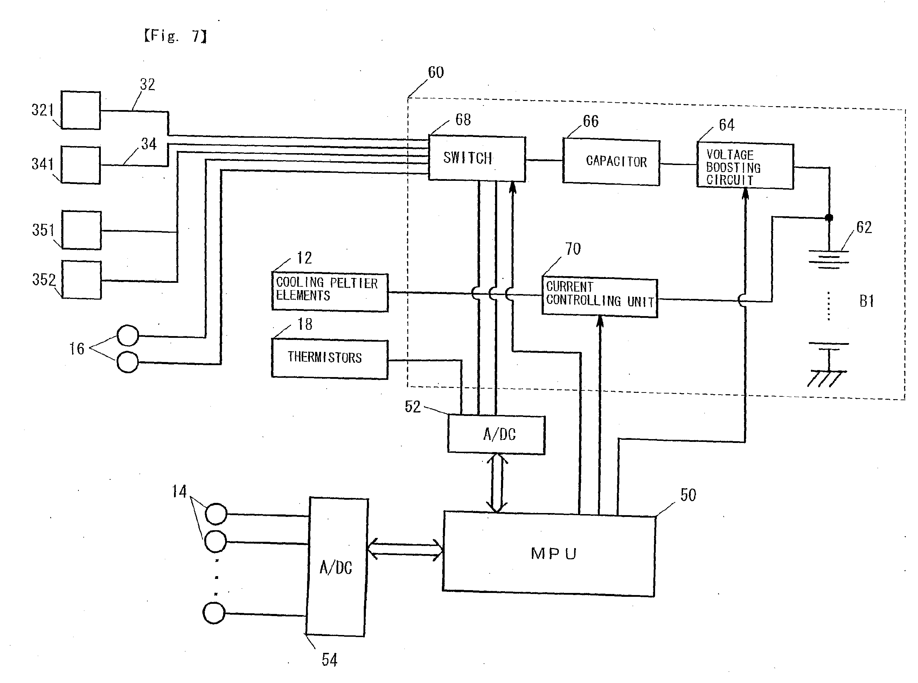

It is also possible to provide separate potential sensors 14 and stimulating electrodes 16 as shown in Fig. 6. In this arrangement, a circuit configuration of the apparatus should be as shown in Fig. 7. The stimulating electrodes 16 are controlled by the switch 68, and the potential sensors 14 are directly input to an A/D converter 54. The pulse voltage/current is applied between the two stimulating electrodes 16.
Also in this arrangement, the pulse voltage/current may be applied between the coil electrodes 351, 352 of the ventricular lead 34, positioned in the right ventricle and the superior vena cava (or right atrium), and the two stimulating electrodes 16. Alternatively, the pulse voltage/current may be applied between the two stimulating electrodes 16, and between one of the two stimulating electrodes 16 and the coil electrodes 351, 352 of the ventricular lead 34, positioned in the right ventricle and the superior vena cava (or right atrium). Also, the pulse voltage/current may be applied between the two stimulating electrodes 16 and the case of main unit 20.

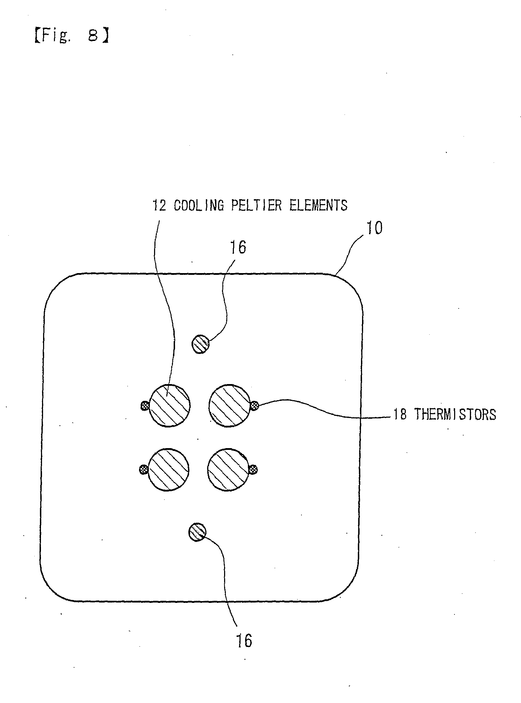

Also as shown in Fig. 8, it is also possible to detect the electrocardiac signals only with the atrial lead 32 and the ventricular lead 34, and to provide the substrate 10 only with the stimulating electrodes 16, eliminating the potential sensors 14. In this arrangement, the pulse voltage/current can be applied according to the exemplary embodiment shown in Fig. 6.

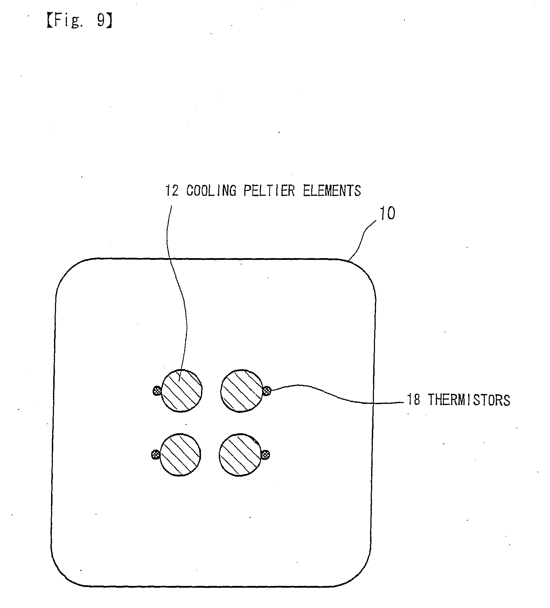

Furthermore, as shown in Fig. 9, it is also possible only to add a cooling unit for cooling the heart regionally to the known ICD. Only the Peltier elements 12 and the thermistors 18 are arranged on the substrate 10, as shown in Fig. 9. In this arrangement, the electrocardiac signals are detected by the atrial lead 32 and the ventricular lead 34, and the pulse voltage/current for stopping ventricular tachycardia or ventricular fibrillation is applied therethorough.

As described above, in this exemplary embodiment of the present invention, if ventricular fibrillation or ventricular tachycardia is detected, the cooling unit is driven to cool the ventricle, and the pulse voltage/current is applied to the cooled region, eliminating a reentrant spiral-wave effectively and allowing ventricular tachycardia to stop. In this manner, development into ventricular fibrillation can be avoided. Because the apparatus applies the pulse voltage/current after the heart is cooled, the applied voltage can be reduced, reducing the sense of discomfort and anxiety of the person implanted with the apparatus upon application of the pulse voltage/current. Therefore, the QOL of the patient can be improved. Also because a lower voltage/current can be used if the pulse voltage/current is applied to the cooled region, damage to the heart can be minimized.

### EMBODIMENT 2

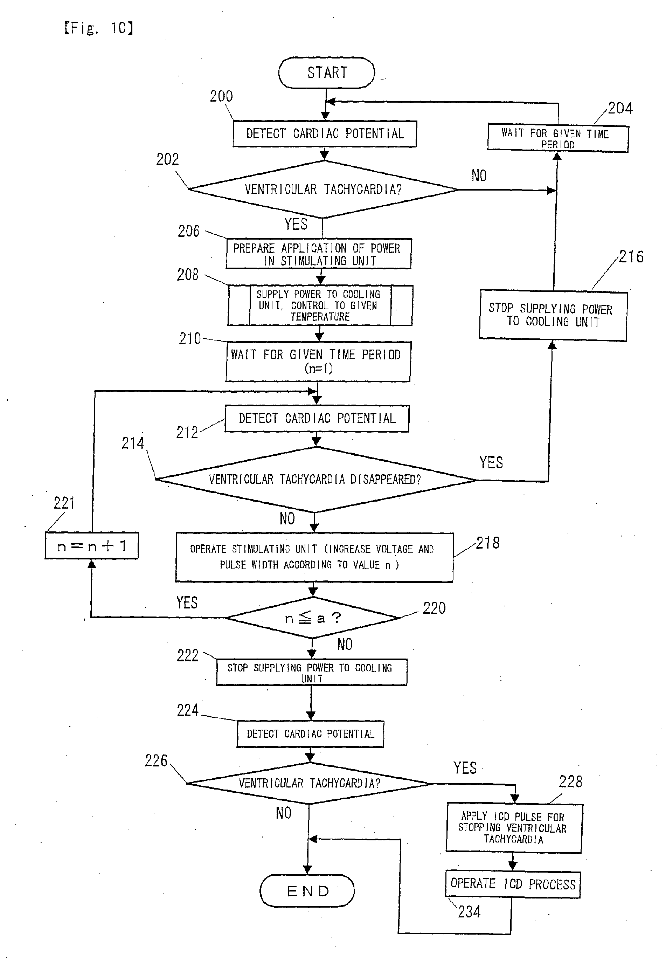

Apparatus structures shown in Figs. 6 and 7 are used in a second exemplary embodiment of the present invention. Procedures executed by the MPU 50 are as shown in Fig. 10. The present apparatus is an implantable fibrillation preventing apparatus designed to prevent ventricular fibrillation upon detection of ventricular tachycardia, a precursor phenomenon leading to ventricular fibrillation. The apparatus according to the first exemplary embodiment operates its fibrillation stopping function upon detection of ventricular tachycardia as well as ventricular fibrillation. The apparatus according to this exemplary embodiment functions to stop ventricular tachycardia upon detection thereof. Steps 200 to 226 correspond to the steps 100 to 126 of Fig. 4. If ventricular tachycardia is detected as in the shown manner, the cooling unit is driven at first to cool the ventricle, and subsequently, the pulse voltage/current is applied to the cooled region after a given time has elapsed, eliminating a reentrant spiral-wave effectively, enabling ventricular tachycardia to stop. In this manner, development into ventricular fibrillation can be avoided. A patient implanted with the known ICD experiences a great anxiety upon defibrillation using a strong pulse voltage/current, resulting in serious impairment of QOL. In contrast, because the apparatus does not apply a strong voltage/current, the anxiety can be reduced and QOL of the patient can be improved.

### EMBODIMENT 3

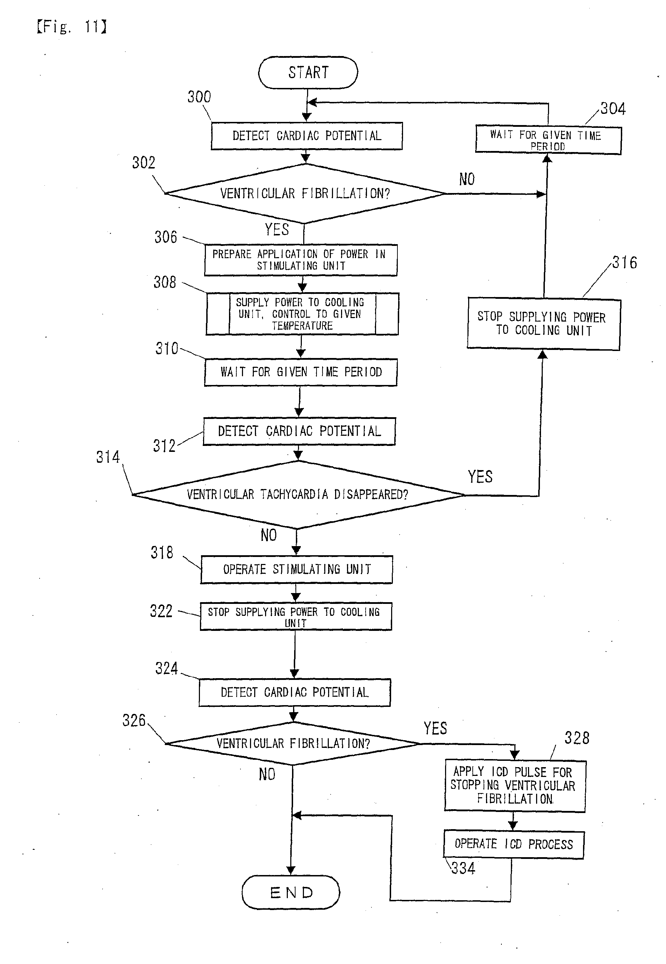

The apparatus may alternatively perform procedures shown in Fig. 11 upon detection of ventricular fibrillation. In other words, the apparatus can execute the procedures according to the second exemplary embodiment shown in Fig. 10 upon detection of ventricular tachycardia, and perform procedures shown in Fig. 11 upon detection of ventricular fibrillation. Steps 300 to 326 correspond to the steps 200 to 226. However, in an adopted method, the voltage of the pulse current/voltage is not gradually increased after the heart cooling upon detection of ventricular fibrillation, unlike the procedures executed upon detection of ventricular tachycardia, but a pulse voltage/current of a given voltage and pulse width is applied to the cooled region one time. This is because the fibrillation must be stopped as soon as possible upon detection thereof. If ventricular fibrillation still persists, at the steps 328 and 334, a pulse voltage/current of a high voltage is applied to the heart via the coil electrodes 351, 352 of the ventricle lead 34, positioned in the right ventricle and the superior vena cava (or right atrium). At this time, the pulse voltage/current may alternatively be applied via the stimulating electrodes 16 arranged on the substrate 10.

The present invention is also effective as a treatment method for preventing a fibrillation, by cooling the heart upon detection of a precursor phenomenon (ventricular tachycardia or a reentrant spiral-wave) of ventricular fibrillation, or by applying a pulse voltage/current after a given time has elapsed since the cooling was started and the reentrant spiral-wave is localized around the cooled region.

Furthermore, the present invention is also effective as a treatment method for stopping a fibrillation by cooling the heart upon detection of ventricular fibrillation, or by applying a relatively low pulse voltage/current after cooling the heart.

### EMBODIMENT 4

This exemplary embodiment of the present invention relates to an implantable defibrillating apparatus. The defibrillating apparatus is mounted on the heart 30 in the same manner as in the first exemplary embodiment as shown in Fig. 1. In this exemplary embodiment, the substrate 10 in Fig. 1 has the structure shown in Fig. 8. In other words, four cooling Peltier elements 12 (cooling units) are placed at the center of the substrate 10. Each Peltier element 12 has a thermistor 18 (temperature detecting unit) at a point on the circumference thereof to detect temperature. On the surface of the substrate 10, the stimulating electrodes 16 are provided to apply the pulse voltage/current to the cooled region. The Peltier elements 12, the thermistors 18 and the stimulating electrodes 16 are fixed in close contact with the epicardium of the left cardiac ventricle.

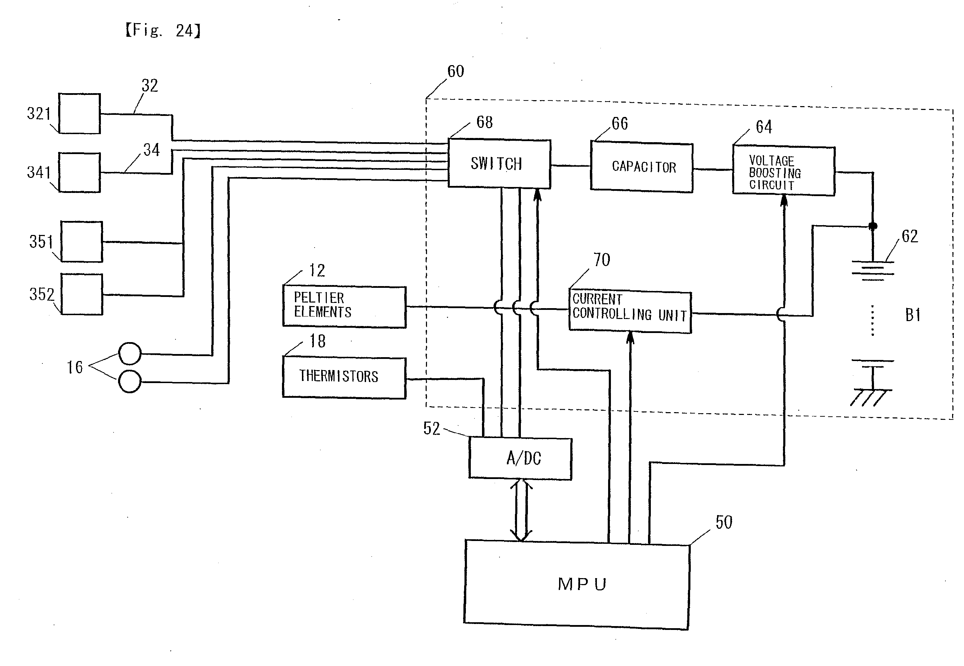

The main unit 20 is implanted subcutaneously in precordial region of a person. The main unit 20 controls conduction of electricity to the stimulating electrodes 16 provided on the substrate 10, receives the electrocardiac signals from the atrial lead 32 and the ventricular lead 34, and controls the pulse voltage/current applied through the coil electrodes 351, 352 of the ventricular lead 34, arranged in the right ventricle and the superior vena cava (or the right atrium).
An electrical configuration of the defibrillating apparatus according to the exemplary embodiment is shown in Fig. 24. Units having the same function as those in the first exemplary embodiment are given the same reference numbers. The controlling unit mainly includes the MPU (microprocessor unit) 50 and the A/D converters 52. The MPU 50 detects electrocardiac signals, processes the detected signals and controls conduction of electricity.

The stimulating unit 60 mainly includes the battery 62, the voltage boosting circuit 64 for boosting up the voltage from the battery 62 on the basis of an instruction received from the MPU 50, the capacitor 66 for storing the electric charge boosted by the voltage boosting circuit 64, the switch 68 for controlling conductivity of input and output terminals and switching thereof, and a current controlling unit 70 for controlling current conduction to the Peltier elements 12 on the basis of an instruction received from the MPU 50 and the stimulating electrodes 16 for applying the pulse voltage/current to the heart. The controlling unit and the stimulating unit described above are incorporated into the main unit 20.

The switch 68 controls switching of a transmission system in a circuit described below. Upon receiving an instruction from the MPU 50, the switch 68 is switched to output the electrocardiac signals, received from the electrode 321 attached at the tip of the atrial lead 32 and the electrode 341 attached at the tip of ventricular lead 34, to the A/D converter 52. The switch 68 is also switched according to the instruction from the MPU 50 so as to output the voltage from the capacitor 66 to the coil electrodes 351, 352 of the ventricular lead 34, respectively positioned in the right ventricle and the superior vena cava (or right atrium).

The switch 68 is also switched according an instruction from the MPU 50 to output the voltage in the capacitor 66 to the stimulating electrodes 16 arranged on the substrate 10.

Signals output from the thermistors 18 are read into the MPU 50 via the A/D converter 52. The current controlling unit 70 operates under control of the MPU 50 to supply electricity to the Peltier elements 12, changing cooling capability thereof; in this manner, heart temperature is controlled to bring the temperature detected by the thermistors 18 to a target temperature.

The electrocardiac signal detecting unit according to the exemplary embodiment of the present invention mainly includes the atrial lead 32, the electrode 321 located at the tip thereof, the ventricular lead 34 and the electrode 341 located at the tip thereof. The controlling unit mainly includes the MPU 50, and A/D converter 52 and the thermistors 18. The cooling unit includes the Peltier elements 12. The stimulating unit includes the battery 62, the voltage boosting circuit 64, the capacitor 66, the switch 68, the stimulating electrodes 16 and the current controlling unit 70.

Operation of the defibrillating apparatus according to the exemplary embodiment is same as that according to the third exemplary embodiment of the present invention shown in Fig. 11. Explanation is provided below with reference to Fig. 11.
At a step 300, the switch 68 is switched so as to be input with the signals from the atrial lead 32 and the ventricular lead 34. In this manner, the potentials at the myocardiums in the right atrium 31 and the right ventricle 37 are detected. These signals are electrocardiac signals representing myocardial excitations. At a step 302, these electrocardiac signals are analyzed to determine whether ventricular fibrillation has occurred or not. It is determined whether it is ventricular fibrillation or not basically on the basis of the excitation cycle (ventricular rate) and duration thereof. It is important not to misconceive a supraventricular arrhythmia, such as sinus tachycardia, atrial fibrillation or flutter, as ventricular fibrillation.

Here, presence of ventricular fibrillation is determined on the basis of cycles and duration of the electrocardiac signals received from the electrode 341, located at the tip of the right ventricular lead 34. Also, presence of supraventricular arrhythmia, such as sinus tachycardia, atrial fibrillation or flutter, is determined on the basis of the electrocardiac signals received from the electrode 321 located at the tip of the right atrial lead 32. When such supraventricular arrhythmias are present, they are distinguished from ventricular fibrillation, and the apparatus is not driven. A fibrillating-detecting algorithm in the arrhythmia-detecting algorithms used in the known ICD may be adopted.

If ventricular fibrillation is not detected, the apparatus is not driven; and the system control proceeds to a step 304, and returns to the step 300 after a given wait time. The electrocardiac signals of the heart are continuously monitored by looping the steps 300-302-304.

If ventricular fibrillation is detected at the step 302, a control signal is output to the voltage boosting circuit 64, and the capacitor 66 is charged to a given voltage. In other words, preparation for application of the pulse voltage/current to the heart takes place in advance. The voltage/current used here is lower than that used in the known ICD.

Subsequently, the system control proceeds to a step 308, where power is supplied to the Peltier elements 12, which is a cooling unit, and a control signal is output to the current controlling unit 70 to reduce the regional temperature of the left ventricular epicardium from the body temperature by 5°C. Upon doing so, temperature detection signals from the thermistors 18 are input, and the power supplied from the current controlling unit 70 is controlled by feedback thereby to bring the detected temperature to the target temperature. By way of this control, the regional temperature of the left ventricular epicardium 38 reaches the specified target.

At the step 310, after the system control has waited until the epicardial temperature reaches the specified temperature, a voltage boosting parameter n is set to 1 initially, and the system control proceeds to a step 312, at which the cardiac potential is detected by each sensor, in the exact same manner as at the step 300. Subsequently, the system control proceeds to a step 314 where it is determined whether ventricular fibrillation has terminated; if it has terminated, the system control proceeds to a step 316 where the power supply to the Peltier elements 12 is stopped. In the manner described above, the system control of the MPU 50 returns to the step 300, and the heart monitoring loop including the steps 300-302-304 is executed.

At the step 314, if it is determined that ventricular fibrillation has not terminated, the system control proceeds to a step 318, at which the stimulating electrodes 16 are connected to the stimulating unit 60, and the switch 56 is switched so as to enable application of the pulse voltage/current. Then, the stimulating unit 60 is operated to apply the pulse voltage/current to the stimulating electrodes 16. Voltage of biphasic waveform, as shown in Fig. 5, is used as a voltage of the pulse voltage/current. The voltage/current is applied between the two stimulating electrodes 16, and the pulse current flows in the cooled region in the left ventricle.

At a step 322, application of the power to the Peltier elements 12 is stopped. At a step 324, the cardiac potential is again detected by the atrial lead 32 and the ventricular lead 34. Subsequently at a step 326, if ventricular fibrillation is detected, it means that ventricular fibrillation could not be terminated by application of the low pulse voltage/current. Therefore, the system control proceeds to a step 328, and the pulse voltage/current used for stopping ventricular fibrillation in the known ICD is applied. The high pulse voltage/current is applied between the coil electrodes 351, 352 of the ventricular lead 34, positioned in the right ventricle and the superior vena cava (or right atrium), and the ICD main unit. In other words, if ventricular fibrillation cannot be eliminated using the method in which the low pulse voltage/current is applied to the cooled region, the high voltage/current is applied between the ventricle and the case of ICD main unit, in the same manner as in the known ICD. Subsequently, processes of the known ICD take place at a step 334, and the system control of the apparatus ends when control ending conditions of the known ICD are established. Then, the system control is started again from the step 300.

In the above exemplary embodiment of the present invention, the pulse voltage/current is applied between the two stimulating electrodes 16; however, the pulse voltage/current may also be applied between the coil electrodes 351, 352 attached to the ventricular lead 34, positioned in the right ventricle and the superior vena cava (or right atrium) and the two stimulating electrodes 16. Alternatively, combination thereof may be used. In other words, it is possible to equalize the potential of one of the two stimulating electrodes 16 and that of the coil electrodes 351, 352 of the ventricular lead 34, positioned in the right ventricle and the superior vena cava (or right atrium), and to apply the pulse voltage/current between these and the other stimulating electrode 16. Furthermore, it is also possible to apply the pulse voltage/current between the stimulating electrodes 16 and the case of main unit 20.

### EMBODIMENT 5

This exemplary embodiment is characterized in that only the cooling unit is added to the conventional implantable ICD. In other words, the substrate 10 includes only the Peltier elements 12 and the thermistors 18. In this arrangement, the electrocardiac signals are detected by the atrial lead 32 and the ventricular lead 34, and the pulse voltage/current for removing stopping ventricular fibrillation is applied via the coil electrodes 351, 352 of the ventricular lead 34, positioned the right ventricle and the superior vena cava (or right atrium). Main portions of the electrical configuration of the apparatus are same as that according to the fourth exemplary embodiment shown in Fig. 24 except for the substrate 10 lacking the stimulating electrodes 16. Also the controlling method is same as the procedure shown in Fig. 11. An exception is that the low pulse voltage/current is applied at first between the case of main unit 20, including the controlling unit and the stimulating unit, and the coil electrodes 351, 352 of the ventricular lead 34, positioned in the right ventricle and the superior vena cava (or right atrium) at the step 318. If the low pulse voltage/current cannot terminate ventricular fibrillation, the high pulse voltage/current, used in the known ICD, is applied between the case of ICD main unit 20 and the coil electrodes 351, 352 of the ventricular lead 34, positioned in the right ventricle and the superior vena cava (or right atrium).

As disclosed above, according to the fifth exemplary embodiment, it is only a matter of fixing the substrate 10 with the cooling unit additionally provided to the known ICD onto the surface of the ventricle, and modifying the procedures of the controlling unit.

### EMBODIMENT 6

An implantable defibrillating apparatus explained below also functions as a fibrillation preventing apparatus for preventing an occurrence of ventricular fibrillation. A sixth exemplary embodiment modifies the fibrillation preventing apparatus according to the first exemplary embodiment to an implantable defibrillating apparatus having a cooling unit. Therefore, the structure according to the sixth exemplary embodiment is identical to the fibrillating apparatus according to the first exemplary embodiment renamed as a defibrillating apparatus. Therefore, the structures according to the first exemplary embodiment shown in Figs. 1 to 9 may also appliy to this exemplary embodiment.

### EMBODIMENT 7

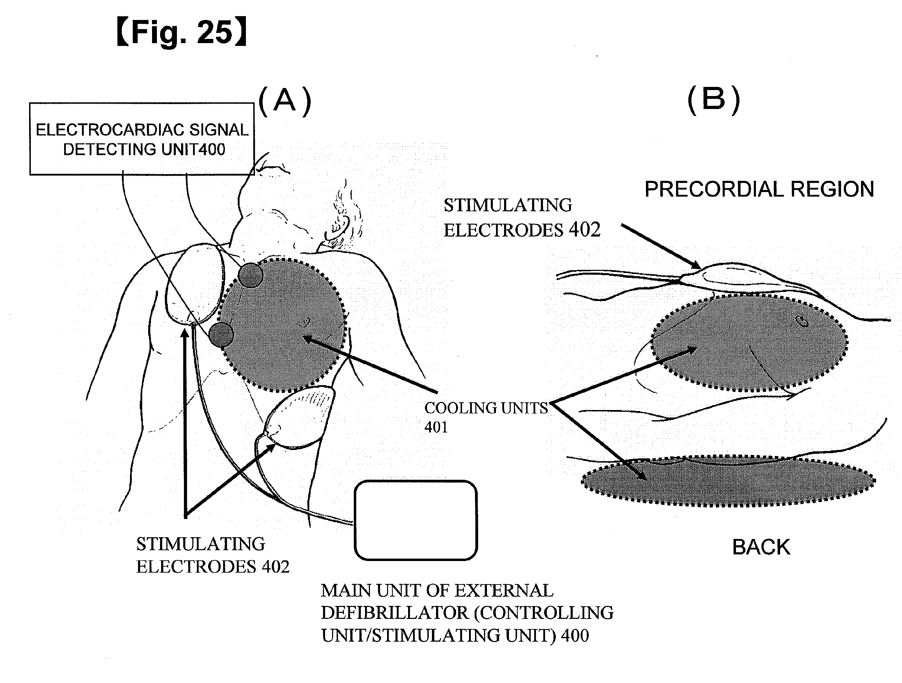

A seventh exemplary embodiment of the present invention is an external defibrillating apparatus having a unit for cooling the heart. It is also possible to include an external defibrillating apparatus having a main unit 400 (the controlling unit and the stimulating unit) of a common external defibrillator, stimulating electrodes 402 included in the controlling unit and a cooling unit for cooling the heart. This apparatus has the structure of the commonly-used external defibrillator with an addition of the cooling unit. A general structure of the apparatus according to this exemplary embodiment is shown in Fig. 25. As shown in panel A of Fig. 25, the electrocardiac signals are detected from the body surface using an electrocardiac signal detecting unit 404. After an operator confirms ventricular fibrillation in an electrocardiographic monitor, the operator attempts to defibrillate a few times by applying a direct current from the body surface (precordial region) using the ordinary external defibrillator main unit (the controlling unit and the stimulating unit) and the stimulating electrodes 402 included in the stimulating unit. The stimulating electrodes 402 are held in contact to body surface at the precordial region, sandwiching the heart.

If the defibrillation is unsuccessful, the operator attaches a cooling unit 401 using cooling media to the back and the precordial region as shown in panel B of Fig. 25, cools the heart, and subsequently applies the direct current again from the precordial region via stimulating electrodes 402. One of the reasons why ordinary defibrillation fails is that multiple reentrant spiral-waves coexist and their frequent breakup occurs one after another. By adopting the method to cool the heart, electric properties of the myocardium in the cooled region are changed (the refractory period is prolonged). The reentrant spiral-waves are expected to be localized around the cooled region and destabilized in favor of their self-termination. Under these conditions, electrical defibrillation efficiency can be improved.

As explained above, because defibrillation efficiency improvement can be expected by cooling using the cooling unit 401, the external defibrillating apparatus according to the present invention includes such a unit constructed by only a cooling unit used for this purpose. Furthermore, the external defibrillating apparatus according to the present invention includes a unit including the cooling unit 401 combined with the stimulating electrodes 402 and the main unit 400 of the external defibrillator.

### EMBODIMENT 8

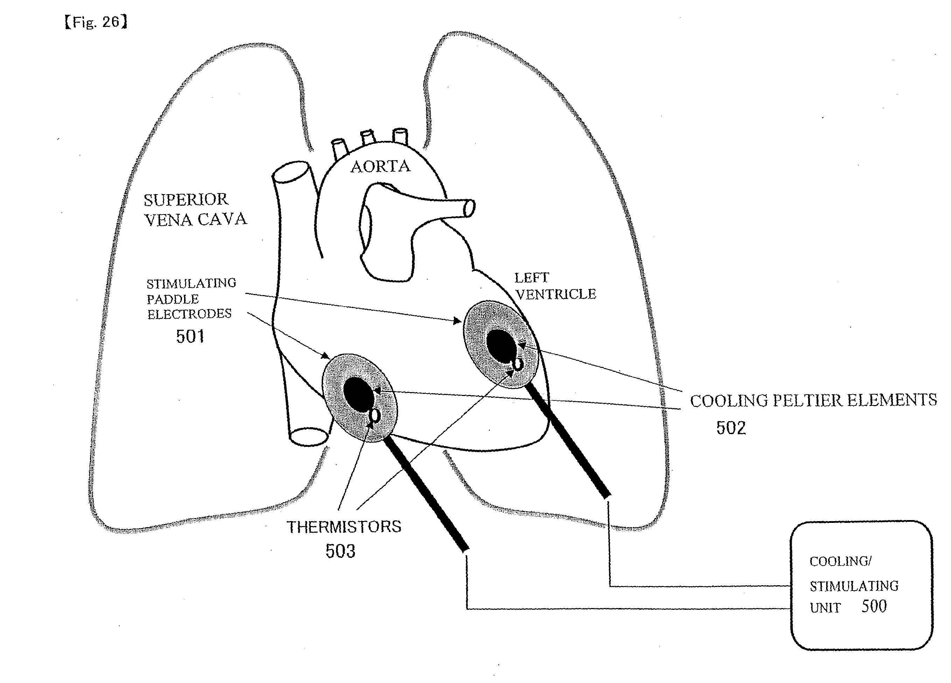

An apparatus according to this exemplary embodiment of the present invention is an external defibrillating apparatus having a function to apply a direct current for defibrillation from the heart surface, and a function to cool the heart. A general structure of the apparatus according to this exemplary embodiment is shown in Fig. 26. The external defibrillating apparatus mainly includes stimulating paddle electrodes 501 with cooling Peltier elements 502 and thermistors 503, and cooling/stimulating units 500. The cooling/stimulating units 500 function to cool the heart by supplying power to the cooling Peltier elements 502, and monitors the myocardial temperature on the basis of the input signal received from the thermistors 503 provided to measure the temperatures, and adjust the cooling function thereof. The cooling/stimulating units 500 also functions to apply direct current to the heart surface.

If electrocardiograms from a body surface indicate ventricular fibrillation and if a few attempts of the ordinary external defibrillation are unsuccessful, a doctor (an operator) may operate an emergency thoracotomy to expose the heart, held the stimulating paddle electrodes 501 to contact with the heart surface while giving a cardiac massage, and attempt to stop ventricular fibrillation by applying a direct current therefrom. If the defibrillation by direct current application from the heart surface fails, the temperature at the heart surface is reduced moderately immediately before subsequent direct current application using the stimulating paddle electrodes 501 having a cooling function and the cooling/stimulating unit 500. Upon confirming that the regional temperature of the heart surface, measured by the thermistors 503, is reduced to the temperature from the body temperature by 5°C. The operator applies a direct current to the heart to terminate the ventricular fibrillation. Because of the reasons described above for the seventh exemplary embodiment, the electrical defibrillating efficiency can be improved.

As an alternative to those apparatuses explained above for the several exemplary embodiments, the present invention is also effective as treatment methods for stopping the fibrillation by cooling the heart upon the occurrence of ventricular fibrillation, or by applying a relatively weak pulse voltage/current after cooling.

Furthermore, the present invention is effective as treatment procedure for preventing ventricular fibrillation by applying global or regional cooling to the heart upon detection of a precursor phenomenon (ventricular tachycardia or a reentrant spiral-wave), and then by applying a pulse voltage/current after a given time or when the reentrant spiral-wave becomes localized around the cooled region.

### Industrial Applicability

The present invention is extremely effective as medical equipment because it can prevent ventricular fibrillation in patients with cardiac diseases or of high risk for ventricular fibrillation. The present invention is also extremely effective as a defibrillator because ventricular fibrillation can be stopped effectively even when a fibrillation occurs. Furthermore, the present invention is extremely effective because it can stop ventricular fibrillation effectively upon the occurrence thereof, and also prevent ventricular fibrillation from occurring.

## Claims

1. A fibrillation preventing apparatus for preventing an occurrence of ventricular fibrillation comprising:
an electrocardiac signal detecting unit for detecting electrocardiac signals, which are electrical potentials of a heart;
a cooling unit for cooling the heart; and
a controlling unit for driving the cooling unit to cool the heart upon detection of a precursor phenomenon that could lead to ventricular fibrillation on the basis of the electrocardiac signals detected by the electrocardiac signal detecting unit.

2. A fibrillation preventing apparatus according to claim 1, wherein the precursor phenomenon is ventricular tachycardia.

3. A fibrillation preventing apparatus according to claim 1, wherein the precursor phenomenon includes a phenomenon on the basis of the occurrence of a reentrant spiral-wave.

4. A fibrillation preventing apparatus according to any one of claims 1 to 3, further including a stimulating unit for applying a pulse voltage/current to the heart, wherein the controlling unit drives the stimulating unit after a given time has elapsed since the cooling unit is driven.

5. A fibrillation preventing apparatus according to any one of claims 1 to 3, further including a stimulating unit that applies a pulse voltage/current to the heart, wherein the controlling unit detects the rotation center of a reentrant spiral-wave appearing in the cooled region of the heart after driving the cooling unit, and drives the stimulating unit on the basis of the detection.

6. A fibrillation preventing apparatus according to any one of claims 1 to 3, further including a stimulating unit that applies a pulse voltage/current to the heart, wherein the controlling unit drives the stimulating unit if the precursor phenomenon has not disappeared after a given time has elapsed since the cooling unit is driven.

7. A fibrillation preventing apparatus according to claim 2, further including a stimulating unit that applies a pulse voltage/current to the heart, wherein the controlling unit drives the stimulating unit if ventricular tachycardia has not terminated after a given time has elapsed since the cooling unit is driven.

8. A fibrillation preventing apparatus according to any one of claims 4 to 7, wherein the pulse voltage/current is applied to a region being cooled by the cooling unit.

9. A fibrillation preventing apparatus according to any one of claims 4 to 8, wherein the pulse voltage/current is applied to a cardiac ventricle or a cardiac atrium.

10. A fibrillation preventing apparatus according to any one of claims 1 to 9, wherein the cooling unit comprises a Peltier element to which a current is applied from the controlling unit.

11. A defibrillating apparatus including a cooling unit that provides reversible cooling of the heart.

12. A defibrillating apparatus according to claim 11 further including a controlling unit for driving and controlling the cooling unit to cool the heart upon detection of ventricular fibrillation.

13. A defibrillating apparatus according to any one of claims 11 or 12 further comprising an electrocardiac signal detecting unit for detecting electrocardiac signals of a heart.

14. A defibrillating apparatus according to claim 13, wherein the electrocardiac signal detecting unit comprises an atrial lead inserted into a cardiac atrium and a ventricular lead inserted into a cardiac ventricle.

15. A defibrillating apparatus according to claim 13 further comprising electrocardiogram recording electrodes attached to a body surface of a patient.

16. A defibrillating apparatus according to claim 12, wherein ventricular fibrillation is detected by way of electrocardiac signals detected by an atrial lead inserted into a cardiac atrium and a ventricular lead inserted into a cardiac ventricle.

17. A defibrillating apparatus according to any one of claims 11 to 16 further including a stimulating unit for applying a pulse voltage/current to the heart.

18. A defibrillating apparatus according to claim 17, wherein the stimulating unit is driven to apply a pulse voltage/current to the heart after the heart is cooled by the cooling unit.

19. A defibrillating apparatus according to claim 17 or 18 further including stimulating electrodes for applying a pulse voltage/current to the heart region cooled by the cooling unit.

20. A defibrillating apparatus according to claim 19, wherein the stimulating electrodes are provided at multiple regions on the heart surface.

21. A defibrillating apparatus according to claim 17 or 18 further comprising stimulating electrodes provided in the right ventricle, and the superior vena cava or the right atrium for applying a pulse voltage/current.

22. A defibrillating apparatus according to claim 17 or 18 further comprising stimulating electrodes attached to a body surface of a patient for applying the pulse voltage/current.

23. A defibrillating apparatus according to claim 12, wherein ventricular fibrillation is detected by way of electrocardiac signals detected by electrocardiogram recording electrodes attached to a body surface of a patient.

24. A defibrillating apparatus according to claim 12, wherein the pulse voltage/current is applied when ventricular fibrillation has not terminated.

25. A defibrillating apparatus according to any one of claims 17 to 24, wherein the pulse voltage/current is applied to the region cooled by the cooling unit.
